(19) Europäisches Patentamt
European Patent Office
Office européen des brevets

(11) Veröffentlichungsnummer: **0 275 523**
**B1**

(12) # EUROPÄISCHE PATENTSCHRIFT

(45) Veröffentlichungstag der Patentschrift: **06.02.91**

(21) Anmeldenummer: **87118931.2**

(22) Anmeldetag: **21.12.87**

(51) Int. Cl.[5]: **C 07 D 307/32,** C 07 B 57/00, A 01 N 43/08

(54) **Optisch aktives Tetrahydro-2-furanon-Derivat als Mikrobizid.**

(30) Priorität: **23.12.86 CH 5201/86**
**18.11.87 CH 4475/87**

(43) Veröffentlichungstag der Anmeldung:
**27.07.88 Patentblatt 88/30**

(45) Bekanntmachung des Hinweises auf die Patenterteilung:
**06.02.91 Patentblatt 91/06**

(84) Benannte Vertragsstaaten:
**AT BE CH DE ES FR GB GR IT LI LU NL SE**

(56) Entgegenhaltungen:
**EP-A-0 077 755**
**GB-A-1 577 702**

(73) Patentinhaber: **CIBA-GEIGY AG**
**Klybeckstrasse 141**
**CH-4002 Basel (CH)**

(72) Erfinder: **Eckhardt, Wolfgang. Dr.**
**Breslauerstrasse 14**
**D-7850 Lörrach (DE)**
Erfinder: **Francotte, Eric, Dr.**
**Violaweg 73/10**
**CH-4303 Kaiseraugst (CH)**
Erfinder: **Kunz, Walter, Dr.**
**Buchenstrasse 9**
**CH-4104 Oberwil (CH)**
Erfinder: **Hubele, Adolf, Dr.**
**Obere Egg 9**
**CH-4312 Magden (CH)**

(74) Vertreter: **Zumstein, Fritz, Dr. et al**
**Bräuhausstrasse 4**
**D-8000 München 2 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 275 523 B1

**Beschreibung**

Die vorliegende Erfindung betrifft das optisch aktive (aS, 1'R)-3-[N-(Methoxyacetyl)-N-(3-chlor-2,6-dimethylphenyl)]-amino-tetrahydro-2-furanon, Verfahren zu seiner Herstellung, mikrobizide Mittel, die es als aktive Komponenten enthalten, sowie die Verwendung dieser Mittel als Mikrobizide im Pflanzenschutz.

Das 3-[N-(Methoxyacetyl)-N-(3-chlor-2,6-dimethylphenyl)]-amino-tetrahydro-2-furanon ist aus der DE—OS 2,804,299 oder aus der entsprechenden GB—PS 1,577,702 als eine mikrobizid wirksame Verbindung bekannt. Nach dieser Veröffentlichung weist die Verbindung der Formel I

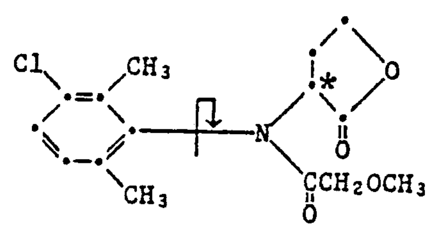

(I)

als Strukturmerkmal ein Asymmetriezentrum an der angedeuteten Stelle* auf. Sie kann als Racemat auf übliche Art in optische Antipoden gespalten werden, wobei die verschiedenen Konfigurationen unterschiedlich starke mikrobizide Wirkung aufweisen.

Es findet sich in dieser Referenz weder ein Hinweis, wie gross diese Wirkungsdifferenz sein mag, noch welcher der beiden Antipoden überhaupt die stärkere mikrobizide Wirkung besitzt. Darüberhinaus werden in der DE—OS 2,804,299 bzw. in der GB—PS 1,577,702 im Rahmen des dortigen chemischen Umfangs keine Angaben zu weiteren Isomerie-Möglichkeiten ausserhalb des Furanonrings gemacht.

Im Falle der konventionell als Racemat erhältlichen Verbindung der Formel I liegt aber nicht nur aufgrund der asymmetrischen Substitution am markierten *C-Atom ein einziges Enantiomerenpaar vor, sondern ein Gemisch von 4 Isomeren, d.h. von zwei diastereomeren Enantiomerenpaaren.

Der Grund hierfür liegt darin, dass das Molekül ausser dem erwähnten.

Asymmetriezentrum noch eine, durch die chirale Achse (⌐↓) bedingte Rotationsisomerie (Atropisomerie) aufweist, welche durch die asymmetrische Chlorsubstitution des rotationsbehinderten 2,6-Dimethylphenylrings verursacht wird. Das in der DE—OS 2,804,299 als Verbindung Nr. 30 genannte racemische 3-[N-(Methoxyacetyl)-N-(3-chlor-2,6-dimethylphenyl)]-amino-tetrahydro-2-furanon erweist sich als ein 1:1-Gemisch, bestehend aus zwei diastereomeren Enantiomerenparren, die bei 140° bzw. 115°C schmelzen und die sich voneinander trennen lassen. Als Trennverfahren eignet sich beispielsweise die Adsorptionschromatographie. Beide Enantiomerenpaare können als Racemate auf übliche Art in optische Antipoden gespalten werden. Aus dem bei 140°C schmelzenden, als Nebenprodukt anfallenden (aS,1'S)(aR,1'R)-Enantiomerenpaar können die Enantiomeren (aS,1'S)- und (aR,1'R)-3-[N-(Methoxyacetyl)-N-(3-chlor-2,6-dimethylphenyl)]-amino-tetrahydro-2-furanone, aus dem bei 115°C schmelzenden erwünschten (aR,1'S)(aS,1'R)-Enantiomerenpaar die Enantiomeren (aR,1'S)- und (aS,1'R)-3-[N-(Methoxyacetyl)-N-(3-chlor-2,6-dimethylphenyl)]-amino-tetrahydro-2-furanone isoliert werden, beispielsweise durch Chromatographie über eine optisch aktive Phase.

Die ungefähre 1:1-Zusammensetzung des nach DE—OS 2,804,299 erhältlichen diastereomeren Enantiomeren-Gemisches schwankt zwischen 45 und 55%.

Es wurde nun gefunden, dass des enantiomere (aS,1'R)-3-[N-(Methoxyacetyl)-N-(3-chlor-2,6-dimethylphenyl)]-amino-tetrahydro-2-furanon eine überraschend stark erhöhte mikrobizide Aktivität verbunden mit einer unerwartet hohen Wirkungsdauer gegenüber dem Isomeren-Gemisch der Formel I aufweist.

Gegenstand vorliegender Erfindung ist daher auch eine Verbindung der Formel I mit einem höheren Anteil am (aS,1'R)-Enantiomeren als 25 Gewichtsprozent, wie es nach der Theorie beim Vorliegen eines racemischen Gemischs von 2 diastereomeren Enantiomerenpaaren zu erwarten wäre. Gegenstand vorliegender Erfindung ist im engeren Sinne eine Verbindung der Formel I mit einem (aS,1'R)-Enantiomeren-Anteil von mindestens 28 Gew.-%, bevorzugt von mindestens 40 Gew.-%, besonders bevorzugt von mindestens 50 Gew.-%, Ganz besonders bevorzugt für praktische Zwecke als Pflanzenfungizide ist eine Verbindung der Formel I mit einem (aS,1'R)-Enantiomeren-Anteil von mindestens 70 Gew.-%, insbesondere von mindestens 90 Gew.-%. Die Erfindung betrifft auch das reine (aS,1'R)-Enantiomere des 3-[N-(Methoxyacetyl)-N-(3-chlor-2,6-dimethylphenyl)]-amino-tetrahydro-2-furanon.

Auch das raceme (aR,1'S)(aS,1'R)-3-[N-(Methoxyacetyl)-N-(3-chlor-2,6-dimethylphenyl)]-amino-tetrahydro-2-furanon weist eine wesentliche Erhöhung der mikrobiziden Aktivität und der Wirkungsdauer gegenüber dem Isomeren-Gemisch der Formel I auf, wobei das Ausmass der Wirkungssteigerung geringer ist als beim (aS,1'R)-Enantiomeren.

Als Pflanzenfungizid ist das Enantiomerenpaar (aR,1'S)(aS,1'R) bzw. sind ungefähre 1:1 Mischungen dieser beiden Anteile (45:55 bis 55:45) besonders geeignet.

Des raceme (aR,1'S)(aS,1'R)-Isomere bildet daher ebenfalls einen Gegenstand der vorliegenden Erfindung.

Die Herstellung des enantiomeren (aS,1'R)-3-[N-(Methoxyacetyl)-N-(3-chlor-2,6-dimethylphenyl)]-amino-tetrahydro-2-furanons kann durch zweimalige chromatographische Trennung des Isomeren-

Gemisches der Formel I und des daraus erhaltenen, bei 115°C schmelzenden (aR,1'S)(aS,1'R)-3-[N-(Methoxyacetyl)-N-(3-chlor-2,6-dimethylphenyl)]-amino-tetrahydro-2-furanon erfolgen.

Der erste Schritt der Auftrennung des aus zwei diastereomeren Enantiomerenpaaren bestehenden Racemats erfolgt an einer stationären festen Phase mit Hilfe einer flüssigen Phase (= Laufmittel), wobei das gewünschte (aR,1'S)(aS,1'R)-Enantiomerenpaar gewonnen wird. Für diesen Trennungsschritt lassen sich alle dem Fachmann geläufigen Trägermaterialien wie Kieselgel, $Al_2O_3$, oder organische Polymer-Harze (Polyamid, Polyacrylamid und dergleichen), ferner Agarose, Sepharose, besonders aber modifizierte Cellulose verwenden. Besonders geeignet ist acetylierte oder benzoylierte Cellulose. Das (aR,1'S)(aS,1'R)-Enantiomerenpaar lässt sich dann als solches in formulierter Form als Pflanzenfugizid verwenden oder weiter auftrennen.

Beim zweiten Trennungsschritt geht man so vor, dass mann die Lösung des Racemats des (aR,1'S)- und (aS,1'R)-3-[N-(Methoxyacetyl)-N-(3-chlor-2,6-dimethylphenyl)]-amino-tetrahydro-2-furanons über eine optisch aktive Phase in die Enantiomeren trennt und das aS,1'R-3-[N-(Methoxyacetyl)-N-(3-chlor-2,6-dimethylphenyl)]-amino-tetrahydro-2-furanon aus der lösung isoliert.

Als optisch aktive feste Phase lässt sich beispielsweise acylierte Cellulose verwenden wie acetylierte oder benzoylierte Cellulose. Beispiele sind Triacetylcellulose oder Tri-(3-methylbenzoyl)cellulose.

Nach einer bevorzugten Variante des beschriebenen Trennverfahrens können alle vier Enantiomeren des nach DE—OS 2,804,299 erhältlichen Isomeren-Gemisches in nur einer Trennstufe anstatt zwei voneinander getrennt werden. Bei dieser Variante wird die verdünnte Lösung des zu trennenden Gemisches in Hexan/Isopropanol (1:1) auf modifizierte Cellulose als optisch aktive Phase aufgetragen und mit einer geeigneten mobilen Phase (= Laufmittel) nacheinander abgelöst. Vorzugsweise wird acylierte (z.B. acetylierte oder benzoylierte Cellulose, insbesondere Tri-(3-methylbenzoyl)-cellulose als stationäre Phase aufgetragen und anschliessend mit einem Hexan/Isopropanol-Gemisch (1:1 bis 9:1) eluiert. Die Lösungen der abgelösten Isomeren werden in Fraktionen aufgefangen und die Isomeren nach Verdampfen des Lösungsmittelgemisches als Verdampfungsrückstand isoliert.

Nach einer ebenfalls bevorzugten Variante des gemischilderten Herstellungsverfahrens kann das bei der Trennung des Isomeren-Gemisches als Nebenprodukt erhältliche, bei 140°C schmelzende (aS,1'S)(aR,1'R)-Enantiomerenpaar wieder zum Ausgangsgemisch racemisiert werden. Solche Racemisierung oder Isomerisierung kann beispielsweise thermisch, durch Erwärmung des (aS,1'S)(aR,1'R)-Enantiomerenpaares auf 140—180°C in der Schmelze, vorzugsweise in Gegenwart eines Tetraalkylammonium-Salzes, z.B. Tetrabutylammoniumbromid, bis zur Einstellung des Gleichgewichts erfolgen. Das resultierende, wieder aus zwei diastereomeren Enantiomerenpaaren bestehende Produkt kann in das Trennverfahren zurückgeführt werden. Dieses zusätzliche Re-Isomerisierungsverfahren bildet ebenfalls einen Gegenstand der vorliegenden Erfindung.

Auch in Racemisierung des (aR,1'S)-3-[N-(Methoxyacetyl)-N-(3-chlor-2,6-dimethylphenyl)]-amino-tetrahydro-2-furanons durch Oeffnung des Laktonringes mit Alkali und durch den anschliessenden unter geeigneten Bedingungen erfolgenden Ringschluss, ist möglich. Das hieraus resultierende Racemat kann gleichfalls in das Trennverfahren zurückgeführt werden.

Die absolute Konfiguration der Enantiomeren wurde durch Röntgenstrukturanalyse ermittelt und lässt sich wie folgt wiedergeben:

(aS,1'S)-Enantiomer)

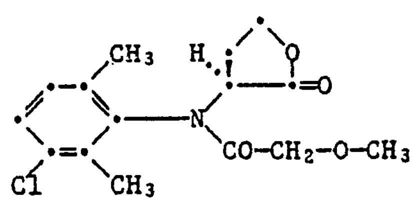

(aR,1'R)-Enantiomer)

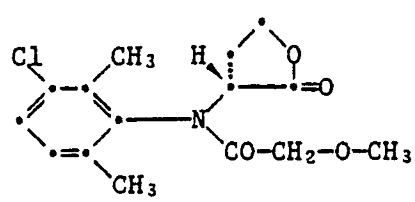

(aR,1'S)-Enantiomer)

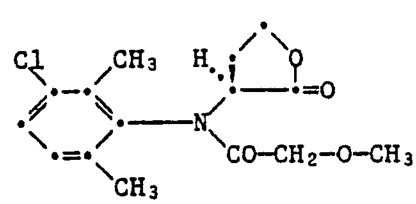

(aS,1'R)-Enantiomer)

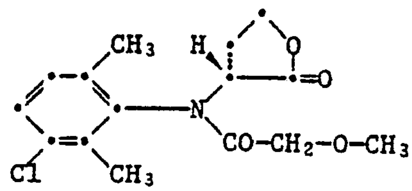

Gegenstand vorliegender Erfindung ist auch ein Verfahren zur Gewinnung von (aR,1'R)(aS,1'R)-3-[N-(Methoxyacetyl)-N-(3-chlor-2,6-dimethylphenyl)]-amino-tetrahydro-2-furanon, das gleichfalls eine wesentlich höhere Fungizid-Wirkung als das aus der DE—OS 2,804,299 bekanntgewordene Racemat entfaltet. Als Pflanzenfungizid ist dieses Diastereomerenpaar im 1:1-Verhältnis oder im ungefähren 1:1-Verhältnis (45:55 bis 55:45) besonders geeignet. Es its gleichfalls ein Gegenstand vorliegender Erfindung.

Beim Herstellungsverfahren wendet man kombiniert a) ein Trennungsverfahren zweier Isomeren eines Zwischenproduktsund b) ein nachfolgendes Chlorierungsverfahren gemäss folgendem Schema an:

a)

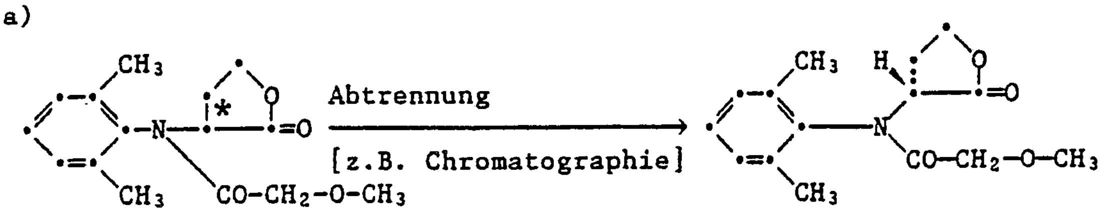


(Racemat) (1'R)-Enantiomer

b) (1'R)-Enantiomer

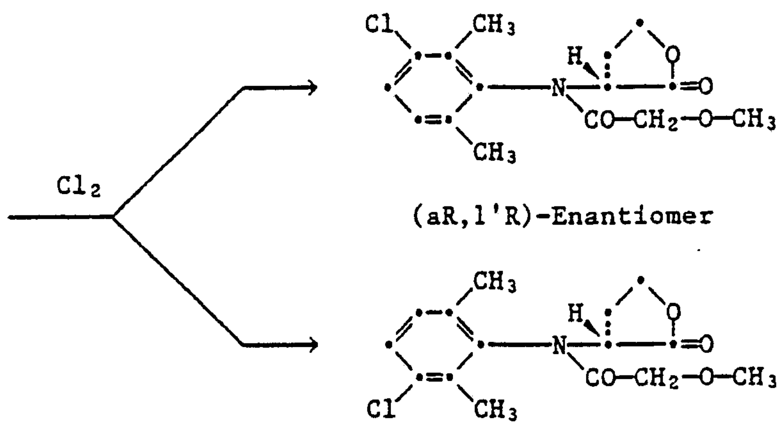


(aR,1'R)-Enantiomer

(aS,1'R)-Enantiomer

Das mit der Chlorierung anfallende Gemisch des (aR,1'R)- und des (aS,1'R)-Enantiomeren, kann als solches als Fungizid verwendet oder weiterhin zur Gewinnung des (aS,1'R)-Entantiomeren wiederum nach der Methode des einleitenden Trennungsschritts a) chromatographisch getrennt werden.

Das raceme Ausgangsprodukt 3-[N-(Methoxyacetyl)-N-2,6-dimethylphenyl)]-amino-tetrahydro-2-furanon ist gleichfalls in der DE—OS 2,804,299 bzw. in der GB—PS 1,577,702 beschrieben.

Für den vorstehend skizzierten chromatographischen Trennungsschritt a) lassen sich alle dem Fachmann geläufigen Trägermaterialien wie Kieselgel, $Al_2O_3$, oder organische Polymer-Harze (Polyamid, Polyacrylamid und dergleichen), ferner Agarose, Sepharose, besonders aber modifizierte Cellulose verwenden. Besonders geeignet ist acetylierte oder benzoylierte Cellulose.

Die Chlorierung des (1'R)-3-[N-(Methoxyacetyl)-N-2,6-dimethylphenyl)]-amino-tetrahydro-2-furanons wird vorteilhaft in einem inerten Lösungsmittel vorgenommen. Als Lösungsmittel sind chlorierte aromatische und aliphatische Kohlenwasserstoffe, wie Chlorbenzol, Methylenchlorid, Chloroform, Kohlenstofftetrachlorid und andere geeignet. Insbesondere sind niedere Alkancarbonsäuren verwendbar, z.B. Ameisensäure, Essigsäure, Propionsäure. Ein bevorzugtes Lösungsmittel ist Ameisensäure.

Die Chlorierung wird vorteilhaft im Temperaturbereich von 0° bis 50°C durchgeführt, z.B. bei Raumtemperatur.

Es ist ferner vorteilhaft, die Chlorierung in Gegenwart von Lewis-Säuren, wie Aluminiumchlorid, Zinkchlorid, Eisen(III)chlorid und Zinn(IV)chlorid durchzuführen. Eine bevorzugte Lewis-Säure ist Eisen(III)-chlorid. Die Lewis-Säuren werden in einer Menge von 1—8 Gew.-%, bezogen auf das zu chlorierende (1'R)-Isomere, eingesetzt. Es kann auch ohne Lewis-Säuren chloriert werden. Die Chlorierung wird bei Normaldruck oder leicht erhöhtem Druck durchgeführt. Für die Chlorierung wird Sulfurylchlorid, bevorzugt aber Chlor verwendet.

Durch das erfindungsgemässe Verfahren a) der Abtrennung des (1'R)-Enantiomeren aus dem racemischen 3-[N-(Methoxyacetyl)-N-(2,6-dimethylphenyl)]-amino-tetrahydro-2-furanon an einer festen Trägerphase (Adsorptionschromatographie) und b) der Chlorierung zu einem (aR,1'R)(aS,1'R)-Isomeren-Gemisch zweier Atropisomerer gelangt man in hoher Ausbeute überraschend zu einer Wirkstoff-Komponente, die eine Fungizid-Wirkung in der gleichen Grössenordnung zeigt wie das hockwirksame Enantiomere (aS,1'R)-3-[N-(Methoxyacetyl)-N-(3-chlor-2,6-dimethylphenyl)]-amino-tetrahydro-2-furanon.

Es wurde gefunden, dass vor allem das (aS,1'R)-3-[N-(Methoxyacetyl)-N-(3-chlor-2,6-dimethylphenyl)]-amino-tetrahydro-2-furanon, aber auch das raceme (aR,1'S)(aS,1'R)-3-[N-(Methoxyacetyl)-N-(3-chlor-2,6-dimethylphenyl)]-amino-tetrahydro-2-furanon und das Isomeren-Gemisch (aR,1'R)- und (aS,1'R)-3-[N-(Methoxyacetyl)-N-(3-chlor-2,6-dimethylphenyl)]-amino-tetrahydro-2-furanon dem aus der DE—OS 2,804,299 bekannt gewordenen Racemat, das 4 Isomeren enthält, in der Mikrobizid-Wirkung um ein Mehrfaches überlegen sind. Diese beiden Isomeren-Gemische und das (aS,1'R)-Enantiomere besitzen jeweils ein für die praktischen Bedürfnisse sehr günstiges Mikrobizid-Spektrum zum Schutze von Kulturpflanzen, ohne diese durch unerwünschte Nebenwirkungen nachteilig zu beeinflussen. Kulturpflanzen seien im Rahmen vorliegender Erfindung beispielsweise Getreide, Mais, Reis, Gemüse, Zuckerrüben, Soja, Erdnüsse, Obstbäume, Zierpflanzen, vor allem aber Reben, Hopfen, Gurkengewächse (Gurken, Kürbis, Melonen), Solanaceen wie Kartoffeln, Tabak und Tomaten, sowie auch Bananen-, Kakao- und Natrukautschuk-Gewächse.

Das (aS,1'R)-Enantiomere oder seine vorstehend genannten Isomeren-Gemische können an Pflanzen oder Pflanzenteilen (Früchte, Blüten, Laubwerk, Stengel, Knollen, Wurzeln) dieser und vewandter Nutzkulturen die auftretenden Pilze eindämmen oder vernichten, wobei auch später zuwachsende Pflanzenteile von derartigen Pilzen verschont bleiben. Die Wirkstoffe sind gegen die den folgenden Klassen angehörenden phytopathogenen Pilze wirksam: Ascomycetes (z.B. Erysiphaceae); Basidiomycetes wie vor allem Rostpilze; Fungi imperfecti (z.B. Moniliales); dann aber besonders gegen die der Klasse der Phycomycetes angehörenden Oomycetes wie Phytophthora, Peronospora, Pseudoperonospora, Pythium oder Plasmopara. Ueberdies wirken die Isomeren systemisch. Sie können ferner als Beizmittel zur Behandlung von Sattgut (Früchte, Knollen, Körner) und Pflanzenstecklingen zum Schutz vor Pilzinfektionen sowie gegen im Erdboden auftretende phytopahtogene Pilze eingesetzt werden.

Als Beispiele, die keinen das Anwendungsgebiet limitierenden Charakter im Rahmen dieser Erfindung besitzten, seien folgende Pflanzen und ihre Agrarprodukte genannt: Getreide (wie Weizen, Gerste, Roggen, Hafer, Reis, Sorghum und Verwandte); Rüben (wie Möhren, Zucker- und Fetterrüben); Kern-, Stein- und Beerenobst (wie Aepfel, Birnen, Pflaumen, Pfirsiche, Mandeln, Kirschen, Erd-, Him- und Brombeeren); Hülsenfrüchte (wie Bohnen, Linsen, Erbsen, Soja); Oelkulturen (wie Raps, Senf, Mohn, Oliven, Sonnenblumen, Kokos, Rizinus, Kakao, Erdnüsse); Gurkengewächse (wie Kürbis, Gurken, Melonen); Fasergewächse (wie Baumwolle, Flachs, Hanf, Jute, Nessel); Citrusflächte; Gemüsesorten (wie Spinat, Salat, Spargel, Kohlarten), Zwiebeln, Tomaten, Kartoffeln, Paprika); Lorbeergewächse (wie Avocadom Cinnamonum, Kampfer) oder Mais, Tabak, Nüsse, Kaffee, Zuckerrohr, Tee, Weintrauben, Kastenien, Hopfen, Bananen, Ananas, Gras (z.B. Golfrasen) und Heu.

Die erfindungsgemässen Wirkstoffe werden üblicherweise in Form von Zusammensetzungen verwendet und können gleichzeitig oder nacheinander mit weiteren Wirkstoffen auf die zu behandelnde Fläche, Pflanze oder Substrat gegeben werden. Diese weiteren Wirkstoffe können sowhol Düngemittel, Spurenelement-Vermittler oder andere das Pflanzenwachstum beeinflussende Präparate sein. Es können aber auch selektive Herbizide, Insektizide, Fungizide, Bakterizide, Nematizide, Molluskizide oder Gemischse mehrerer dieser Präparate sein, zusammen mit gegebenenfalls weiteren in der Formulierungstechnik üblichen Trägerstoffen, Tensiden oder anderen applikationsfördernden Zusätzen.

Geeignete Träger und Zusätze können fest oder flüssig sein und entsprechen den in der Formulierungstechnik zweckdienlichen Stoffen, wie z.B. natürlichen oder regenerierten mineralischen Stoffen, Lösungs-, Dispergier-, Netz-, Haft-, Verdickungs-, Binde- oder Düngemitteln. Besonders vorteilhafte Zusatzstoffe sind Phospholipide.

Ein bevorzugtes Verfahren zum Aufbringen eines erfindungsgemässen Wirkstoffes bzw. eines (agro)-chemischen Mittels, das mindestens einen dieser Wirkstoffe enthält, ist das Aufbringen auf das Blattwerk (Blattapplikation). Anzahl der Applikation und Aufwandmenge richten sich dabei nach dem Befallsdruck für den entsprechenden Erreger (Pilzsorte). Die erfundungsgemässen Wirkstoffe können aber auc über den Erdboden durch das Wurzelwerk in die Pflanze gelangen (systemische Wirkung), indem man den Standort der Pflanze mit einer flüssigen Zubereitung tränkt oder die Substanzen in fester Form in den Boden einbringt, z.B. in Form von Granulat (Bodenapplikation). Die erfindungsgemässen Verbindungen können aber auch auf Samenkörner aufgebracht werden (Coating), indem man die Körner entweder mit einer flüssigen Zubereitung des Wirkstoffs tränkt oder sie mit einer festen Zubereitung beschichtet. Darüber hinaus sind in besonderen Fällen weitere Applikationsarten möglich, so z.B. die gezielte Behandlung der Pflanzenstengel oder der Knospen.

Die erfindungsgemässen Verbindungen werden dabei in unveränderter Form oder vorzugsweise zusammen mit den in der Formulierungstechnik üblichen Hilfsmittel eingesetzt und werden daher z.B. zu Emulsionskonzentraten, streichfähigen Pasten, direkt versprühbaren oder verdünnbaren Lösungen, verdünnten Emulsionen, Spritzpulvern, löslichen Pulvern, Stäubemitteln, Granulaten, durch Verkapselungen in z.B. polymeren Stoffen in bekannter Weise verarbeitet. Die Anwendungsverfahren wie

Versprühen, Vernebeln, Verstäuben, Verstreuen, Bestreichen oder Giessen werden gleich wie die Art der Mittel den angestrebten Zielen und den gegebenen Verhältnissen entsprechend gewählt. Günstige Aufwandmengen liegen in allgemeinen bei 50 g bis 5 kg Aktivsubstanz (AS) je ha; bevorzugt 100 g bis 2 kg AS/ha, insbesondere bei 200 g bis 600 g AS/ha.

Die Formulierungen d.h. die einer erfindungsgemässen Wirkstoff und gegebenenfalls eine festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Ether und Ester, wie Ethanol, Ethylenglykol, Ethylenglykolmonomethyl- oder -ethylether, Ketone wie Cyclohexanon, stark polare Lösungsmittel wie N-Methyl-2-pyrrolidon, Dimethylsulfoxid oder Dimethylformamid, sowie gegebenenfalls epoxydierte Pflanzenöle wie epoxydiertes Kokosnussöl, Sonnenblumenöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kielselsäure oder hochdisperse saugfähige Polymerisate zugesetzt werden. Als gekörnte, adsoprtive Granulatträger kommen poröse Typen wie z.B. Bimsstein, Ziegelbruch, Sepiolit oder Bentonit, als nicht adsorptive Trägermaterialien z.B. Calcit oder Sand in Frage. Darüber hinaus kann eine Vielzahl von vorgranulierten Materialien anorganischer oder organischer Natur wie insbesondere Dolomit oder zerkleinerte Pflanzenrückstände, wie z.B. Korkmehl oder Sägemehl, verwendet werden.

Als oberflächenaktive Verbindungen kommen je nach Art des zu formulierenden erfindungsgemässen Wirkstoffes nichtionosgene, kation-und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen, als auch wasserlösliche synthetische oberflächenaktive Verbindungen sein.

Als Seifen seien die Alkali- oder gegenenfalls substituierten Ammoniumsalze von höheren Fettsäuren ($C_{10}$—$C_{22}$), wie z.B. die Na- oder K-Salze der Oel- oder Stearinsäure, oder von natürlichen Fettsäuregemischen, die z.B. aus Kokosnuss- oder Talgöl gewonnen werde können. Ferner sind als Tenside auch die Fettsäure-methyllaurinsalze sowie modifizierte und nicht modifizierte Phospholipide zu erwähnen.

Häufiger werden jedoch sog. synthetische Tenside verwendet, insbesondere Fettsulfonate, Fettsulfate, sulfonierte Benzimidazolderivate oder Alkylsulfonate.

Als nichtionische Tenside kommen in erster Linie Polyglykoletherderivate von alphatischen oder cycloaliphatischen Alkoholen, gesättigten oder ungesättigten Fettsäuren und Alkylphenolen in Frage, die 3 bis 30 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können.

Ferner kommen auch Fettsäureester von Polyoxyethylsorbitan wie das Polyoxyethylensorbitan-trioleat im Betracht.

Bei den kationischen Tensiden handelt es sich vor allem um quatäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder neidrige Hydroxyalkylreste aufweisen. Die Salze liegen vorzugsweise als Halogenide, Methylsulfate oder Ethylsulfate vor, z.B. das Stearyltrimethylammoniumchlorid oder das Benzyldi(2-chlorethyl)ethylammoniumbromid.

Die in der Formulierungstechnik gebräuchlichen Tenside sind u.a. in folgenden Publikationen beschrieben:

"McCutcheon's Detergents and Emulsifiers Annual" MC Publishing Corp. Ridgewood, New Jersey, 1979;

Dr. Helmut Stache "Tensid Taschenbuch", Carl Hanser Verlag München/Wien 1981.

Auf dem Vorratsschutzgebiet werden die für die menschliche und tierische Ernährung unbedenklichen Zuschlagstoffe bevorzugt.

Die fungiziden Mittel gemäss vorliegender Erfindung enthalten in der Regel 0,1 bis 95 Gew.-% des erfindungsgemässen (aS,1'R)-Enantiomeren in einem höheren Anteil unter den 4 Isomeren der Verbindung der Formel I als dem theoretischen Anteil von 25 Gewichtsprozent entspricht, zusammen mit 99,9 bis 5 Gew.-% eines festen und/oder flüssigen Trägermaterials.

Bevorzugt sind Mittel, die im Wirkstoffanteil 28 Gew.-% des (aS,1'R)-Entantiomeren oder mehr enthalten bzw. daneben einen gleich hohen oder geringeren Anteil des (aR,1'R)-Enantiomeren oder einen gleich hohen oder geringeren Anteil des (aR,1'S)-Entantiomeren besitzen.

Vor allem sind Mittel bevorzugt, die in der Wirkstoffmenge 40 Gew.-% des (aS,1'R)-Enantiomeren oder mehr enthalten, und solche, die in der Wirkstoffmenge daneben noch einen gleich hohen oder geringeren Anteil des (aR,1'R)-Enantiomeren oder einen gleich hohen oder geringeren Anteil des (aR,1'S)-Enantiomeren besitzen.

Besonders bevorzugt sind Mittel, die in der Wirkstoffmenge 50 Gew.-% des (aS,1'R)-Enantiomeren

oder mehr enthalten, und solche, die in der Wirkstoffmenge daneben noch einen 100 Gew.-% ergänzenden oder auch geringeren Anteil des (aR,1'R)-Enantiomeren oder des (aR,1'S)-Enantiomeren besitzen.

Weiterhin sind Mittel bevorzugt, die in der Wirkstoffmenge 70 Gew.-% des (aS,1'R)-Enantiomeren oder mehr enthalten, insbesondere solche, deren Wirkstoffmenge im wesentlichen (= 90 Gew.-% oder mehr) aus dem (aS,1'R)-Enantiomeren besteht.

Die vorstehenden Angaben beziehen sich also auf Mengenverhältnisse der vier Isomeren des 3-[N-(methoxyacetyl)-N-(3-chlor-2,6-dimethylphenyl)]-aminotetrahydro-2-furan, bei denen das (aS,1'R)-Enantiomeren nach Möglichkeit den höchsten Anteil darstellt, anteilsmässig gefolgt von entweder den (aR,1'R)- oder dem (aR,1'S)-Enantiomeren, wobei jedes der sonst verbleibeneden Enantiomeren trotzdem in geringen Anteilen vorhanden sein kann.

Die angegebenen Mengenanteile beziehen sich ausschliesslich auf den Wirkstoff der Formel I und schliessen keine Menge anderer Wirkstoffe ein, die möglicherweise in den erfindungsgemässen Mitteln weiterhin anwesend sein können.

Während als Handelsware eher konzentrierte Mittel bevorzugt werden, verwendet der Endverbraucher in der Regel verdünnte Mittel.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Derartige (agro)chemische Mittel sind ein Bestandteil der vorliegenden Erfindung.

Die nachfolgenden Beispiele dienen der Illustration der Erfindung, ohne dieselbe einzuschränken (Prozente und Teile beziehen sich auf Gewicht; Temperaturen sind in Celsiusgraden angegeben).

Herstellungsbeispiele

1.1 Herstellung des Enantiomerenpaares (aR,1'S)(aS,1'R)-3-[N-(Methoxyacetyl)-N-(3-chlor-2,6-dimethylphenyl)]-amino-tetrahydro-2-furanon und des Enantiomerenpaares (aS,1'S)(aR,1'R)-3-[N-(Methoxyacetyl)-N-(3-*chlor-2,6-dimethylphenyl)]-amino-tetrahydro-2-furanon*

70 g des nach DE—OS 2,804,299 hergestellten Iosmeren-Gemisches von 3-[N-(Methoxyacetyl)-N-(3-chlor-2,6-dimethylphenyl)]-amino-tetrahydro-2-furanon der Formel I werden mittels einer Kieselgel-Flashchromatographiesäule mit Essigsäureäthylester/Diäthylether (1/4) als Fliessmittel aufgetrennt.

Resultat;

33,8 g (aS,1'S)(aR,1'R)-3-[N-(Methoxyacetyl)-N-(3-chlor-2,6-dimethylphenyl)]-amino-tetrahydro-2-furanon, Smp. 140°C,

32,6 g (aR,1'S)(aS,1'R)-3-[N-(Methoxyacetyl)-N-(3-chlor-2,6-dimethylphenyl)]-amino-tetrahydro-2-furanon, Smp. 115°C.

1.2. Herstellung der Enantiomeren (aS,1'S)-3-[N-(Methoxyacetyl)-N-(3-chlor-2,6-dimethylphenyl)]-amino-tetrahydro-2-furanon und (aR,1'R)-3-[N-(Methoxyacetyl)-N-(3-chlor-2,6-dimethylphenyl)]-amino-tetra-hydro-2-furanon

1 g des (aS,1'S)(aR,1'R)-3-[N-(Methoxyacetyl)-N-(3-chlor-2,6-dimethylphenyl)]-amino-tetrahydro-2-furanon werden über eine mit Triacetylcellulose gefüllte Mitteldruckchromatographiesäule bei 2 bar Druck mit einem aus Aethanol (95 Vol%) und Wasser (5 Vol%) bestehenden Fliessmittel in die beiden Enantiomeren aufgetrennt.

Resultat:

0,215 g (aR,1'R)-3-[N-(Methoxyacetyl)-N-(3-chlor-2,6-dimethylphenyl)]-amino-tetrahydro-2-furanon, Smp. 117—120°C, $[\alpha]_D^{24}$ (in $CHCl_3$): $+64 \pm 1°$ (ee >98%),

0,232 g (aS,1'S)-3-[N-(Methoxyacetyl)-N-(3-chlor-2,6-dimethylphenyl)]-amino-tetrahydro-2-furanon, Smp. 115—117°C, $[\alpha]_D^{20}$ (in $CHCl_3$): $-64 \pm 1°$ (ee >99%).

1.3 Herstellung der Enantiomere (aR,1'S)-3-[N-(Methoxyacetyl)-N-(3-chlor-2,6-dimethylphenyl)]-amino-tetrahydro-2-furanon und (aS,1'R)-3-[N-(Methoxyacetyl)-N-(3-chlor-2,6-dimethylphenyl)]-amino-tetra-hydro-2-furanon

0,5 g (aR,1'S)(aS,1'R)-3-[N-(Methoxyacetyl)-N-(3-chlor-2,6-dimethylphenyl)]-amino-tetrahydro-2-furanons werden über eine mit Triacetylcellulose gefüllte Mitteldruckchromatographiesäule bei 2 bar Druck mit einem aus Aethanol (95 Vol%) und Wasser (5 Vol%) bestehenden Fliessmittel in die beiden Enantiomeren aufgetrennt.

Resultat:

0,154 g (aR,1'S)-3-[N-(Methoxyacetyl)-N-(3-chlor-2,6-dimethylphenyl)]-amino-tetrahydro-2-furanon, Smp. 98—100°C, $[\alpha]_D^{20}$, (in $CHCl_3$): $-91,2 \pm 1°$ (ee >95%),

0,152 g des hochwirksamen (aS,1'R)-3-[N-(Methoxyacetyl)-N-(3-chlor-2,6-dimethylphenyl)]-amino-tetrahydro-2-furanon, Smp. 94—96°C, $[\alpha]_D^{20}$, (in $CHCl_3$): $+96 \pm 1°$ (ee >99%).

1.4 Herstellung der einzelnen Isomeren (aS,1'S)-, (aR,1'R)-, (aR,1'S)- und (aS,1'R)-3-[N-(Methoxyacetyl)-N-(3-chlor-2,6-dimethylphenyl)]-amino-tetrahydro-2-furanon in einer Trennstufe aus ihrem Gemisch

Auf eine mit Tri-(3-methylbenzoyl)-cellulose gefüllte HPLC-Säule (0,46 × 25 cm), werden 20 µl einer 1% Lösung des Isomerengemischs in Hexan/2-Propanol (1:1) aufgetragen. Die Chromatographie wird dann bei einem Durchfluss von 1 ml/Min. mit einem aus Hexan und 2-Propanol (60:40) bestehenden Fliessmittel durchgeführt. Unter diesen Bedingungen werden die vier Isomeren getrennt. Die Retentionszeiten der einzelnen Isomeren sind die folgenden:

| Isomere | Retentionszeit (Min.) |
|---|---|
| (aR,1'S) | 21,7 |
| (aS,1'S) | 31,0 |
| (aS,1'R) | 42,5 |
| (aR,1'R) | 67,7 |

Die Isomeren werden nach Verdampfen des Lösungsmittelgemisches und nach Trocknen rein isoliert. (Die physikalischen Daten entsprechen den in den vorangehenden Beispielen angegebenen Werten). Das in die stationäre Phase eingesetzte Tri-(3-methylbenzoyl)-cellulose wird wie folgt zubereitet:

10 g Tri-(3-methylbenzoyl)-cellulose werden in 300 ml Methylenchlorid mit 50 ml Heptanol versetzt. Die erhaltene Lösung wird bei Raumtemperatur in eine bei 400 Upm gerührte 0.7% Natriumlaurylsulfatlösung (240 ml) getropft. Anschliessend wird bei derselben Rührgeschwindigkeit bei 40—42°C (Badtemperatur) das Methylenchlorid abgedampft. Der Rückstand wird abfiltriert und mit Wasser und Ethanol gewaschen. Das pulverige Produkt wird im Vakuumschrank bei 80°C (20 Std.) getrocknet. Ausbeute: 9,6 g (96% der Theorie). Die kugleförmigen mit einem Durchmesser von 10—20 µm können nach Bedarf durch Sieben oder Sedimentieren fraktioniert werden. Weitere physikalische Eigenschaften des Produktes:

Spez. Oberflache: 57,8 $m^2/g$ (nach BET)

Schmelzwärme ΔH: 12,7 J/g.

Die gezielte Herstellung eines (aR,1'R)- und (aS,1'R)-Isomerengemischs von 3-[N-(methoxyacetyl)-N-(3-chlor-2,6-dimethylphenyl)]-amino-tetrahydro-2-furanon durch Chlorierung von (1'R)-3-[N-(Methoxyacetyl)-N-(2,6-dimethylphenyl)]-amino-tetrahydro-2-furanon lässt sich leicht und in hoher Ausbeute erreichen (Beispiel 2.1). Dieses Gemisch kann formuliert als hochwirksames Pflanzen-Mikrobizid eingesetzt werden. Es kann daraus aber auch, sofern gewünscht, das (aS,1'R)-Enantiomere abgetrennt werden (Beispiel 2.2).

Das als Ausgangsprodukt für die Chlorierung zu verwendente (1'R)-3-[N-(methoxyacetyl)-N-(2,6-dimethylphenyl)]-amino-tetrahydro-2-furanon lässt sich etwa nach Art des folgenden Beispiels 2.0 aus dem zugrundeliegenden Racemat abtrennen.

Herstellungsbeispiele

2.0 Auftrennung der enantiomeren Zwischenprodukte (1'S)-3-[N-(Methoxyacetyl)-N-(2,6-dimethylphenyl)]-amino-tetrahydro-2-furanon und (1'R)-3-[N-(Methoxyacetyl)-N-(2,6-dimethylphenyl)]-amino-tetrahydro-2-furanon

2 g 3-[N-(Methoxyacetyl)-N-(2,6-dimethylphenyl)]-amino-tetrahydro-2-furanon werden über eine mit Triacetylcellulose gefüllte Mitteldruckchromatographiesäule bei 2 bar Druck mit einem aus Aethanol (95% Vol%) und Wasser (5 Vol%) bestehenden Fliessmittel in die beiden Enantiomeren aufgetrennt.

Resultat:

0,808 g 1'R-3-[N-(Methoxyacetyl)-N-(2,6-dimethylphenyl)]-amino-tetrahydro-2-furanon, Smp. 63—65°C, $[\alpha]_D^{24}$, (in $CHCl_3$): +84,5 ± 1° (ee >99%),

0.978 g 1'S-3-[N-(Methoxyacetyl)-N-(2,6-dimethylphenyl)]-amino-tetrahydro-2-furanon, Smp. 62—64°C, $[\alpha]_D^{24}$, (in $CHCl_3$): −80,3 ± 1° (ee >95%).

2.1 Herstellung eines ungefähren 1:1-Gemischs von (aR,1'R)- und (aS,1'R)-3-[N-(Methoxyacetyl)-N-(3-chlor-2,6-dimethylphenyl)]-amino-tetrahydro-2-furanon

0,222 g (1'R)-3-[N-(Methoxyacetyl)-N-(2,6-dimethylphenyl)]-amino-tetrahydro-2-furanon der Formel I werden in 30 ml Ameisensäure gelöst. 0,06 g Chlor werden bei Raumtemperatur in Gegenwart einer Spur Eisen(III)chlorid in die Lösung eingeleitet. Die Lösung wird ca. 1 Std. bei Raumtemperatur gerührt und das Lösungsmittel unter vermindertem Druck verdampft. Der Rückstand wird in Essigsäureäthylester gelöst, die Lösung mit Wasser extrahiert, mit Natriumsulfat getrocknet, filtriert und eingedampft. Das getrocknetete Gemisch schmilzt bei 79—82°C, es enthält je ca. 50% der beiden Isomeren, d.h. das Isomerenverhältnis schwankt zwischen 45:55 bis 55:45.

2.2 Herstellung des reinen (aS,1'R)-3-[N-(Methoxyacetyl)-N-(3-chlor-2,6-dimethylphenyl)]-amino-tetrahydro-2-furanon

Der durch Chlorierung gemäss Beispiel 2.1 erhaltene Rückstand wird in Essigsäureäthylester gelöst, die Lösung mit Wasser extrahiert, mit Natriumsulfat getrocknet, filtriert und eingedampft. Das rohe Produktegemisch wird an einer Kieselgel-Flashsäule mit Essigsäureäthylester/Hexan (1/3) als Fliessmittel aufgetrennt.

Resultat;

0,070 g (aR,1'R)-3-[N-(Methoxyacetyl)-N-(3-chlor-2,6-dimethylphenyl)]-amino-tetrahydro-2-furanon, $[\alpha]_D^{20}$, (in $CHCl_3$): +63,8 ± 2°,

0,075 g (aS,1'S)-3-[N-(Methoxyacetyl)-N-(3-chlor-2,6-dimethylphenyl)]-amino-tetrahydro-2-furanon, $[\alpha]_D^{20}$, (in $CHCl_3$): +88 ± 3°.

Formulierungsbeispiele für Wirkstoffe der Formel I einschliesslich Isomeren und deren Gemische, die nach den Beispielen 1.1—1.4 sowie 2.1 und 2.2 hergestellt werden können.

[In Klammern Prozentsatz an (aS,1'R)-Isomeren in Wirkstoff der Formel I]

| 1. Emulsion-Konzentrate | a) | b) | c) |
|---|---|---|---|
| Wirkstoff [70% (aS,1'R)] | 25% | 40% | 50% |
| Ca-Dodecylbenzolsulfonat | 5% | 8% | 6% |
| Ricinusöl-polyäthylenglykoläther (36 Mol Aethylenoxid) | 5% | — | — |
| Tributylphenoyl-polyäthlenglykoläther (30 Mol Aethylenoxid) | — | 12% | 4% |
| Cyclohexanon | — | 15% | 20% |
| Xylolgemisch | 65% | 25% | 20% |

Aus solchen Kozentraten können durch Verdünnen mit Wasser Emulsionen jeder gewünschten Konzentration hergestellt werden.

| 2. Lösungen | a) | b) | c) | d) |
|---|---|---|---|---|
| Wirkstoff [40% (aS,1'R)] | 80% | 10% | 5% | 95% |
| Aethylenglykol-monomethyläther | 20% | — | — | — |
| Polyäthylenglykol M G 400 | — | 70% | — | — |
| N-Methyl-2-pyrrolidon | — | 20% | — | — |
| Epoxydiertes Kokosnussöl | — | — | 1% | 5% |
| Benzin (Siedegrenzen 160—190°C) | — | — | 94% | — |

(MG = Molekulargewicht)

| 3. Granulate | a) | b) |
|---|---|---|
| Wirkstoff [80% (aS,1'R)] | 5% | 10% |
| Kaolin | 94% | — |
| Hochdisperse Kieselsäure | 1% | — |
| Attapulgit | — | 90% |

Der Wirkstoff wird in Methylenchlorid gelöst, auf den Träger aufgesprüht und das Lösungsmittel anschliessend im Vakuum abgedampft. Man erhält Granulate für die Erdbodenapplikation.

4. Stäubemittel

| | a) | b) |
|---|---|---|
| Wirkstoff [50% (aS,1'R)] | 2% | 5% |
| Hochdisperse Kieselsäure | 1% | 5% |
| Talkum | 97% | — |
| Kaolin | — | 90% |

Durch inniges Vermischen auf Trägerstoffe mit dem Wirkstoff erhält man gebrauchsfertige Stäubemittel.

5. Spritzpulver

| | a) | b) | c) |
|---|---|---|---|
| Wirkstoff [92% (aS,1'R)] | 25% | 50% | 75% |
| Na-Ligninsulfonat | 5% | 5% | — |
| Na-Laurylsulfat | 3% | — | 5% |
| Na-Diisobutylnaphthalinsulfonat | — | 6% | 10% |
| Octylphenolpolyäthylenglykoläther (7—8 Mol Aethylenoxid) | — | 2% | — |
| Hockdisperse Kiesellsäure | 5% | 10% | 10% |
| Kaolin | 62% | 27% | — |

Der Wirkstoff wird mit den Zusatzstoffen gut vermischt und in einer geeigneten Mühle vermahlen. Man erhält Spritzpulver, die sich mit Wasser zu Suspensionen jeder gewünschten Konzentration verdünnen lassen.

6. Emulsions-Konzentrat

| | |
|---|---|
| Wirkstoff [28% (aS,1'R)] | 10% |
| Octylphenolpolyäthylenglykoläther (4—5 Mol Aethylenoxid) | 3% |
| Ca-Dodecylbenzolsulfonat | 3% |
| Ricinusölpolyglykoläther (35 Mol Aethylenoxid) | 4% |
| Cyclohexanon | 30% |
| Xylolgemisch | 50% |

Aus diesem Konzentrat können durch Verdünnen mit Wasser Emulsionen jeder gewünschten Konzentration hergestellt werden.

7. Stäubemittel

| | a) | b) |
|---|---|---|
| Wirkstoff [97% (aS,1'R)] | 5% | 8% |
| Talkum | 95% | — |
| Kaolin | — | 92% |

Man erhält anwendungsfertige Stäubemittel, indem der Wirkstoff mit dem Träger vermischt auf einer geeigneten Mühle vermahlen wird.

8. Extruder Granulat

| | |
|---|---|
| Wirkstoff [85% (aS,1'R)] | 10% |
| Na-Ligninsulfonat | 2% |
| Carboxymethycellulose | 1% |
| Kaolin | 87% |

Der Wirkstoff wird mit den Zusatzstoffen vermischt, vermahlen und mit Wasser angefuchtet. Dieses Gemisch wurde extrudiert und anschliessend im Luftstrom getrocknet.

9. Umhüllungs-Granulat

| | |
|---|---|
| Wirkstoff [90% (aS,1'R)] | 3% |
| Polyäthylenglykol M G 200 | 3% |
| Kaolin | 94% |

(MG = Molekulargewicht)

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate, die besonders für Erdbodenapplikationen geeignet sind.

10. Suspensions-Konzentrat

| | |
|---|---|
| Wirkstoff [55% (aS,1'R)] | 40% |
| Aethylenglykol | 10% |
| Nonylphenolpolyäthyleglykoläther (15 Mol Aethylenoxid) | 6% |
| N-Ligninsulfonat | 10% |
| Carboxymethylcellulose | 1% |
| 37%ige wässrige Formaldehyd-Lösung | 0,2% |
| Silikonöl in Form einer 75%igen wässrigen Emulsion | 0,8% |
| Wasser | 32% |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

3. Biologische Beispiele

3.1 *Pythium ultimum* auf *Beta vulgaris* (Zuckerrübe, cv. Kleinwanzleben Monogerm) und auf *Pythium ultimum* auf *Zea mays* (Mais, cv. Sweet Corn)

Testprinzip: Bondenpilz: protektiv lokale Bodenapplikation.

Testmethode: Myzel von *Pythium ultimum* wird mit Erde gemischt (500 ml Myzelsuspension auf 10 Liter Erde) und das Pilz-Erdgemisch in 250 ml Plastikschalen abgefüllt. Nach einer 4-tägigen Inkubation bei 10°C werden pro Schale 10 Körner der zu prüfenden Pflanze (Mais oder Zuckerrube) gesteckt. Am nächsten Tag werden die so vorbereitetn Schalen mit je 50 ml, aus 25% Spritzpulver und Wasser hergestellten Spritzlösungen mit 20; 6; 2; 0,6; 0,2; 0,06 und 0,02 ppm AS übergossen. Nach einer 7-tägigen Inkubationsphase bei 10°C und einer anschliessenden 4-tägigen Inkubationsphase bei 22°C wird die Wirkung der Testsubstanzen durch zahlenmässige Bestimmung des Auflaufs der Testpflanzen nach dem folgenden Notenschema bewertet:

| mikrobizide Aktivität % | Bewertungsnote |
|---|---|
| >95 | 1 |
| 80—95 | 3 |
| 50—80 | 6 |
| 0—50 | 9 (= wirkungslos) |

Die erzielten Resultate sind in der Tabelle I zusammengefasst.

Tabelle 1

| Isomere der Verbindung der Formel I (Wirksubstanz) | Bewertungsnote ** bei einer Dosis der Wirksubstanz ppm | 20 | 6 | 2 | 0,6 | 0,2 | 0,06 |
|---|---|---|---|---|---|---|---|
| 1. Isomeren-Gemisch * | Mais | 1 | 1 | 2 | 6 | 9 | 9 |
| | Rüben | 1 | 1 | 1 | 2 | 8 | 8 |
| 2. Racemat (aS,1'S)(aR,1'R) | Mais | 1 | 3 | 8 | 9 | 9 | 9 |
| | Rüben | 2 | 2 | 8 | 9 | 9 | 9 |
| 3. Racemat (aR,1'S)(aS,1'R) | Mais | 1 | 2 | 3 | 5 | 8 | 9 |
| | Rüben | 1 | 1 | 1 | 1 | 5 | 8 |

* Gemäss DE-OS 2 804 299 bestehend aus 45 % der (aR,1'S)°(aS,1'R) und 55 % der (aS,1'S) (aR,1'R) diastereomeren Enantiomerenpaare der Verbindung der Formel I.

** Bewertungsnote als Mittelwert aus je 3 Parallelversuchen.

Das unter 3 genannte Racemat gemäss vorliegender Erfindung erzielt bis hinab zu einer Dosis von 0,6—0,2 ppm fungizide Wirkung.

3.2 Wirkung gegen Phytophthora infestans auf Tomatenpflanzen

a) Residual-protektive Wirkung

Tomatenpflanzen wurden nach 3-wöchiger Anzucht mit einer aus Spritzpulver des Wirkstoffes hergestellten Spritzbrühe (0,02% Aktivsubstanz) besprüht. Nach 24 Stunden wurden die behandelten Pflanzen mit einer Sporangiensuspension des Pilzes infiziert. Die Beurteilung des Pilzbefalls erfolgte nach einer Inkubation der infizierten Pflanzen während 5 Tagen bei 90—100% Luftfeuchtigkeit und 20°C.

b) Systemische Wirkung

Zu Tomatenpflanzen wurde nach 3-wöchiger Anzucht eine aus Spritzpulver des Wirkstoffes hergestellte Spritzbrühe gegossen [0,002% Aktivsubstanz bezogen auf das Erdvolumen]. Es wurde darauf geachtet, dass die Spritzbrühe nicht mit den oberirdischen Pflazenteilen in Berührung kam. Nach 48 Stunden wurden die behandelten Pflanzen mit einer Sporangiensuspension des Pilzes infiziert. Die Beurteilung des Pilzbefalls erfolgte nach einer Inkubation der infizierten Pflanzen während 5 Tagen bei 90—100% relativer Luftfeuchtigkeit und 20°C.

Die Resultate wurden nach dem unter Beispiel 3.1. beschriebenen Bewertungsschema beurteilt und die zur Erzielung der gleichen Bewertungsnote 1—2 benötigte Dosis jeweils auf die Dosis des aktivsten Enantiomeren aS,1'R bezogen. Die auf diese Weise ermittelten Dosisfaktoren (f) sind in der Tabelle 2 zusammengefasst.

3.3 *Plasmopara viticola auf Reben* (Rebensämlinge, cv. Gutedel (Chasselas) 3 Töpfe mit je 1 Pflanze/ Prüfsubstanz)

a) Residual protektive Blattapplikation

5 Wochen alte Rebensämlinge werden mit einer aus der formulierten Prüfsubstanz hergestellten Spritzbrühe besprüht und 1 Tag später mit einer Sporangiensuspension (200'00 Sprorangien/ml) von *P. viticola* infiziert. Die Inkubation erfolgt bei 20°C in einer Gewächshauskabine; einer 14-stündigen Inkubationsphase mit 100% rel. Luftfeuchtigkeit folgen 4 Tage mit 75—85% und abschliessend zur Induktion der Pilzsporulation noch eine Nacht mit 100%. Nach der 6-tägigen Inkubation wird der Pilzbefall ausgewertet (Bewertungsschema wie Beispiel 3.1).

b) Systemische Wirkung

5 Wochen alte Rebensämlinge werden mit einer aus der formulierten Prüfsubstanz hergestellten Spritzbrühe angegossen. 3 Tage später werden die Pflanzen mit einer Sporensuspension (200'000 Sporangien/ml) von *P. viticola* infiziert. Ausführung des Versuchs wie unter a) beschrieben.

Die analog zum Beispiel 3.2. ermittelten Dosisfaktoren sind in der Tabelle 2 zusammengefasst.

3.4 Systemische Wirkung gegen Pythium debaryanum an Beta vulgaris

Der Pilz wurde auf Karottenschnitzel-Nährlösung kultiviert und einer Erde-Sand-Mischung beigegeben. Die so infizierte Erde wurde in Blumentöpfe abgefüllt und mit Zuckerrübensamen besät. Gleich nach der Aussat wurden die als Spritzpulver formulierten Versuchspräparate als wässrige Suspensionen in die Erde gegossen (20 ppm Wirkstoff bezogen auf das Erdvolumen). Die Töpfe wurden darauf während 3 Wochen im Gewächshaus bei ca. 20°C aufgestellt. Die Erde wurde dabei durch leichtes Ueberbrausen gleichmässig feucht gehalten.

Bei der Auswertung der Tests wurde die Zahl der aufgelaufenen Zuckerrübenpflanzen sowie der Anteil gesunder und kranker Pflanzen bestimmt.

Die analog zum Versuch 3.2. ermittelten Dosisfaktoren sind in der Tabelle 2 zusammengefasst.

3.5 Systemische Wirkung gegen Pythium debaryanum an Zea mays

Der Pilz wurde auf Karottenschnitzel-Nährlösung kultiviert und einer Erde-Sand-Mischung beigegeben. Die so infizierte Erde wurde in Blumentöpfe abgefüllt und mit Maissamen besät. Gleich nach der Aussaat wurden die als Spritzpulver formulierten Versuchspräparate als wässrige Suspensionen in die Erde gegossen (20 ppm Wirkstoff bezogen auf das Erdvolumen). Die Töpfe wurden darauf während 3 Wochen im Gewächshaus bei ca. 20°C aufgestellt. Die Erde wurde dabei durch leichtes Ueberbrausen gleichmässig feucht gehalten. Bei der Auswertung der Tests wurde die Zahl der aufgelaufenen Maispflanzen sowie der Anteil gesunder und kranker Pflanzen bestimmt.

Die analog zum Versuch 3.2. ermittelten Dosisfaktoren sind in der Tabelle 2 zusammengefasst.

Tabelle 2

| Isomere der Verbindung der Formel I (Wirksubstanz) | Dosisfaktoren (f) der Versuche | | | | | |
|---|---|---|---|---|---|---|
| | 3.2 | | 3.3 | | 3.4 | 3.5 |
| | a | b | a | b | | |
| Enantiomer aS,1'R | 1 | <1 | <1 | 1 | 1 | 1 |
| Isomeren-Gemisch* | 3,3 | 1 | <1 | 3,3 | 3,3 | 10 |
| Enantiomer aS,1'S | 3,3 | 3,3 | >3 | 10 | 10 | 3,3 |
| Enantiomer aR,1'R | 33 | 3,3 | >3 | 100 | 10 | 33 |
| Enantiomer aR,1'S | 33 | 3,3 | >3 | 3,3 | 33 | 10 |

Aus der Tabelle 2 ist ersichtlich, dass das (aS,1'R)-Enantiomer gemäss vorliegender Erfindung eine durchschnittlich 3-30fach bessere Wirkung im Vergleich zu den übrigen drei Enantiomeren und zum Racemat (= Isomeren-Gemisch* gemäss DE—OS 2,804,299) besitzt.

3.6 Hinderung des Pilzwachstums in vitro (Agarinkorporationstest)

0,1 ml 0,75%-ige acetonsiche Lösung der Wirksubstanzen wird auf 15 ml Wasser steril verdünnt. Je ein ml aus dieser Stammlösung bzw. aus der daraus durch Wasserzusatz steril hergestellten Verdünnungsreihe werden in 19 ml Nährboden in 9 cm ⌀ Petrischalen bei 50°C inkorporiert. Die Wirksubstanzmengen

betragen 50, 5, 0,5, 0,05 und 0,005 ppm pro Schale. Nach 2 Stunden werden diese Schalen und die wirkstofflosen Kontrollschalen mit Phytophthora capsici in Form von Agarrondellen (0,6 mm ø, bewachsene Seite nach unten) beimpft. Die beimpften Kulturen werden bis zur Bewertung des Wachstums bei 60—70% rel. Feuchtigkeit und bei 18°C in Dunkelheit gestellt. Nach 4—6 Tagen, wenn die Kultur der Kontrollschale 2/3 der Petrischale erfüllt, erfolgt die Auswertung der Versuchsreihe, wobei die Wirkstoffkonzentration gegen das radial ermittelte Wachstum (% des Kontrollversuchs) auf einem Graphikon logarithmisch aufgetragen wird. Zum Vergleich der einzelnen Wirksubstanzen wird jeweils jene Konzentration graphisch ermittelt, bei welcher das Wachstum 50% des Kontrollversuchs erreicht ($EL_{50}$-Wert). Die $EL_{50}$-Werte sind in der Tabelle 3 zusammengefasst:

Tabelle 3

| Isomere der Verbindung der Formel I (Wirksubstanz) | $EL_{50}$ bei ppm WS/Schale |
|---|---|
| Enantiomer aS,1'R | 0,011 |
| Diastereomer (aR,1'R)(aS,1'R) | 0,011 |
| Isomeren-Gemisch* | 0,064 |
| Enantiomer aR,1'S | 0,26 |
| Enantiomer aS,1'S | 0,62 |
| Enantiomer aR,1'R | 2,7 |

Versuch 3.6 beweist, dass das hochwirksame (aS,1'R)-Enantiomer im ca. 1:1 Gemsich mit den (aR,1'R)-Enantiomer eine gleich hohe Wirksamkeit erzielt.

3.7 Beispiel zur Bestimmung der Dauerwirkung als Bondenfungizid

Die zu prüfenden Wirkstoffsubstanzen werden als Spritzpulver, jeweils mit 100 ml Wasser verrührt und mit 20 Liter Erde vermischt. Die Konzentration der eingesetzten Aktivsubstanzen wird auf trockene Erde bezogen.

In steriler Erde aufgezogenene Tabakpflazen (Hicks-Sorte) werden im 5—6 Blattzustand in fungizidhaltige Ende (in 5-Liter-Töpfen) ausgepflanzt. Zwei Tage danach werden die Furchen auf beiden Seiten der Pflanze durch Inokulation mit insgesamt zweimal 20 ml einer Sporen-Suspension (enthaltend 10000 Sporangien/ml) von Phytophthora nicotianae künstlich infiziert.

Die Bewertung erfolgt in regelmässigen Abständen mit Noten (N) gemäss einer Skala von 1—100 unter Berücksichtigung der Welkens der Blätter, der Stengel-Schädigung und der Intensität des Schadens. Bei vollstädigem Schutz ist N = 0.

Bis zur Erscheinung der ersten typischen Krankheitszeichen gilt die Pflanze als geschützt. Die Prüfung dauert 100 Tage, während derer die Tabakpflanze reift.

Die Daurerwirkung der einzelnen Wirkstoffe wird mit Hilfe von Faktoren verglichen. Hierzu wird für die einzelnen bei verschiedenen Konzentrationen (ppm) eingesetzten Wirkstoffe jene Wirkungsdauer in Tagen ermittelt, bei welcher der Schutz (N) dem des Isomeren-Gemisches* (Definition siehe Tabelle 1) bei 0,25 ppm Konzentration nach 50 Tagen entspricht. Der Vergleichsfaktor (f) wird berechnet nach folgender Formel

$$f = \frac{0,25}{\text{Konz. (ppm)}} \cdot \frac{\text{Dauer (Tage)}}{50}$$

Die für die einzelnen Wirksubstanzen so ermittelten Faktoren wurden in der Tabelle 4 zusammengefasst, aus der die hohe Dauerwirkung des (aS,1'R)-Enantiomeren und des (aR,1'S)(aS,1'R)-Enantiomerenpaars im Vergleich zu den anderen drei Enantiomeren und zu zwei Gemischen hervorgeht.

Tabelle 4

| Isomere der Verbindung der Formel I (Wirksubstanz) | Faktor Dauerwirkung |
|---|---|
| aR,1'R | 0,025 |
| (aS,1'S)(aR,1'R) | 0,17 |
| aR,1'S | 0,17 |
| aS,1'S | 0,25 |
| Isomeren-Gemisch* | 1 |
| (aR,1'S)(aS,1'R) [Erfindung] | 2 |
| aS,1'R [gem. Erfindung] | 4 |

3.8 Wirkung gegen Phytophthora infestans auf Tomatenpflanzen

a) Residual-protektive Wirkung

b) Systemische Wirkung
Die Testmethodik entspricht der unter Beispiel 3.2 genannten.

3.9 Wirkung gegen Plasmopara viticola auf Reben
a) Residual-protektive Blattapplikation

b) Systemische Wirkung
Die Testmethodik entspricht der unter Beispiel 3.3 genannten.
Es wurden nebeneinander das (aR,1'S)(aS,1'R)-Enantiomerenpaar und das (aR,1'R)(aS,1'R)-Diastereomere in beiden Indikationen geprüft, und ihre Wirkung wurde dem parallel geprüften und aktivsten (aS,1'R)-Enantiomeren gegenübergestellt. In der Tabelle 5 ist nach dem Vorbild der Tabelle 2 angegeben eine um wieviel höhere Aufwandmenge bei dem jeweiligen Diastereomeren eingesetzt werden musste (Dosisfaktor f), um die gleich volle Wirkung (>95%; Note 1) zu erzielen wie das (aS,1'R)-Enantiomere.

Tabelle 5

| Isomere der Verbindung der Formel I (Wirksubstanz) | Dosisfaktor (f) Bsp. 3.8 a) | b) | Bsp. 3.9 a) | b) |
|---|---|---|---|---|
| aS,1'R-Enantiomer | 1 | 1 | 1 | 1 |
| (aR,1'S)(aS,1'R)-Enantiomerenpaar | 3,3 | 3,3 | 1 | 3,3 |
| (aR,1'R)(aS,1'R)-Diastereomer | 1 | 10 | 0,3 | 1 |

Im Gegensatz zu den Resultaten der Tabelle 2, bei denen das (aS,1'R)-Enantiomer als überragendes Aktivitätsprinzip gegenüber den anderen Enantiomeren und gegenüber der vorbekannten racemischen Verbindung (= Isomeren-Gemisch*) erkannt wird, zeigt Tabelle 5 eine überraschend ähnliche Wirkungshöhe des Enantiomerenpaares und des Diastereomeren zu der des (aS,1'R)-Enantiomeren, obwohl das wirksame (aS,1'R)-Enantiomer nur je einen der beiden Anteile stellt. Dieses Resultat deutet auf eine offenbare Wechselwirkung der Enantiomeren hin, die die biologische Aktivität verstärken kann.

**Patentansprüche für die Vetragsstaaten: AT BE CH DE FR GB GR IT LI LU NL SE**

1. Fungizides Mittel enthaltend als Wirkstoff 0,1—95 Gew.-% 3-[N-(Methoxyacetyl)-N-(3-chlor-2,6-dimethylphenyl)]-amino-tetrahydro-2-furanon in ungleichmässiger Verteilung seiner 4 möglichen Isomeren, dadurch gekennzeichnet, dass das aktivitätsbestimmende (aS,1'R)-Enantiomere einen höheren Anteil als 25 Gewichtsprozent darstellt, zusammen mit einem geeigneten Trägermaterial.

2. Mittel gemäss Anspruch 1, wobei der Anteil des (aS,1'R)-Enantiomeren 28 Gewichtsprozent oder mehr beträgt.

3. Mittel gemäss Anspruch 1, wobei der Anteil des (aS,1'R)-Enantiomeren 40 Gewichtsprozent oder mehr beträgt.

4. Mittel gemäss Anspruch 1, wobei der Anteil des (aS,1'R)-Enantiomeren 50 Gewichtsprozent oder mehr beträgt.

5. Mittel gemäss Anspruch 1, wobei der Anteil des (aS,1'R)-Enantiomeren 70 Gewichtsprozent oder mehr beträgt.

6. Mittel gemäss Anspruch 1, wobei das 3-[N-(Methoxyacetyl)-N-(3-chlor-2,6-dimethylphenyl)]-amino-tetrahydro-2-furanon im wesentlichen (= 90 Gew.-% oder mehr) aus dem (aS,1'R)-Enantiomeren besteht.

7. Mittel gemäss Anspruch 1, dadurch gekennzeichnet, dass das aktivitätsbestimmende (aS,1'R)-Enantiomere den höchsten Anteil darstellt, gefolgt von einem gleich hohen oder geringeren Anteil an wahlweise dem (aR,1'R)-Enantiomeren oder dem (aR,1'S)-Enantiomeren.

8. Mittel gemäss Anspruch 7, wobei der Wirkstoff im wesentlichen aus einem Gemisch des (aS,1'R)-Enantiomeren mit dem (aR,1'R)-Enantiomeren besteht.

9. Mittel gemäss Anspruch 7, wobei der Wirkstoff im wesentlichen aus einem Gemisch des (aS,1'R)-Enantiomeren mit dem (aR,1'S)-Enantiomeren besteht.

10. 3-[N-(Methoxyacetyl)-N-(3-chlor-2,6-dimethylphenyl)]-amino-tetrahydro-2-furanon der Formel I

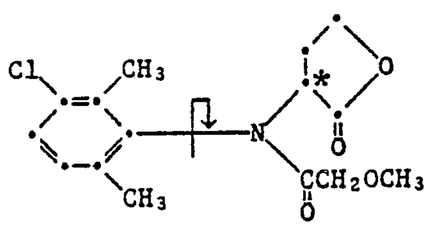

(I)

mit einem höheren Anteil am (aS,1'R)-Enantiomeren als 25 Gewichtsprozent.

11. Die Verbindung der Formel I gemäss Anspruch 10, wobei der Anteil des (aS,1'R)-Enantiomeren mindestens 28 Gewichtsprozent beträgt.

12. Die Verbindung der Formel I gemäss Anspruch 10, wobei der Anteil des (aS,1'R)-Enantiomeren mindestens 40 Gewichtsprozent beträgt.

13. Die Verbindung der Formel I gemäss Anspruch 10, wobei der Anteil des (aS,1'R)-Enantiomeren mindestens 50 Gewichtsprozent beträgt.

14. Die Verbindung der Formel I gemäss Anspruch 10, wobei der Anteil des (aS,1'R)-Enantiomeren mindestens 70 Gewichtsprozent beträgt.

15. Die Verbindung der Formel I gemäss Anspruch 10, wobei der Anteil des (aS,1'R)-Enantiomeren mindestens 90 Gewichtsprozent beträgt.

16. Das (aS,1'R)-Enantiomere der Verbindung 3-[N-(Methoxyacetyl)-N-(3-chlor-2,6-dimethylphenyl)]-amino-tetrahydro-2-furanon.

17. Das Racemat von (aR,1'S)- und (aS,1'R)-3-[N-(Methoxyacetyl)-N-(3-chlor-2,6-dimethylphenyl)]-amino-tetrahydro-2-furanon.

18. Das Diastereomerengemisch (aS,1'R)(aR,1'R) des 3-[N-(Methoxyacetyl)-N-(3-chlor-2,6-dimethylphenyl)]-amino-tetrahydro-2-furanon.

19. Verfahren zur Herstellung des Enantiomerenpaares (aR,1'S)(aS,1'R)-3-[N-(Methoxyacetyl)-N-(3-chlor-2,6-dimethylphenyl)]-amino-tetrahydro-2-furanon, dadurch gekennzeichnet, dass man das entsprechende Racemat, das aus zwei diastereomeren Enantiomerenpaaren besteht, an einer stationären festen Phase mit Hilfe einer flüssigen Phase vom (aS,1'S)(aR,1'R)-Enantiomerenpaar abtrennt.

20. Verfahren gemäss Anspruch 19, wobei die feste Phase ein Trägermaterial auf der Basis von Agarose, Sepharose, Cellulose, Polyamid oder Polyacrylamide ist.

21. Verfahren gemäss Anspruch 20, wobei das Trägermaterial benzoylierte oder acetylierte Cellulose ist.

22. Verfahren gemäss Anspruch 21, wobei Triacetylcellulose als Trägermaterial verwendet wird.

23. Verfahren gemäss Anspruch 19, wobei die feste Phase ein Trägermaterial auf der Basis von Kieselgel oder Aluminiumoxid ist.

24. Verfahren zur Herstellung des enantiomeren (aS,1'R)-3-[N-(Methoxyacetyl)-N-(3-chlor-2,6-dimethylphenyl)]-amino-tetrahydro-2-furanon, dadurch gekennzeichnet, dass man eine Lösung des Racemats bestehend aus (aR,1'S)- und (aS,1'R)-3-[N-(Methoxyacetyl)-N-(3-chlor-2,6-dimethylphenyl)]-amino-tetrahydro-2-furanon chromatographisch über eine optisch aktive feste Phase in die Enantiomeren trennt und as (aS,1'R)-Enantiomere aus der Lösung isoliert.

25. Verfahren gemäss Anspruch 24, wobei als optisch aktive feste Phase Triacetylcellulose verwendet wird.

26. Verfahren gemäss Anspruch 24, wobei als optisch aktive feste Phase Tri-(3-methylbenzoyl)-cellulose verwendet wird.

27. Verfahren zur Herstellung von (aR,1'R)(aS,1'R)-3-[N-(methoxyacetyl)-N-(3-chlor-2,6-dimethylphenyl)]-amino-tetrahydro-2-furanon, dadurch gekennzeichnet, dass man (1'R)-3-[N-(methoxyacetyl)-N-(2,6-dimethylphenyl)]-amino-tetrahydro-2-furanon in einem geeigneten Lösungsmittel bei 0—50°C in Anwesenheit oder Abwesenheit einer Lewis-Säure chloriert.

28. Verfahren zur Herstellung von (aR,1'R)(aS,1'R)-3-[N-(methoxyacetyl)-N-(3-chlor-2,6-dimethylphenyl)]-amino-tetrahydro-2-furanon, dadurch gekennzeichnet, dass man aus racemischen 3-[N-(methoxyacetyl)-N-(2,6-dimethylphenyl)]-amino-tetrahydro-2-furanon a) an einem festen Trägermaterial das (1'R)-Enantiomer abtrennt, und b) das erhaltene (1'R)-Enantiomer in einem geeigneten Lösungsmittel bei 0°—50°C in Anwesenheit oder Abwesenheit einer Lewis-Säure chloriert.

29. Verfahren gemäss Anspruch 28, wobei Silicagel oder $Al_2O_3$ als festes Trägermaterial verwendet wird.

30. Verfahren gemäss Anspruch 28, wobei ein festes Trägermaterial auf Basis von Cellulose verwendet wird.

31. Verfahren gemäss Anspruch 30, wobei acylierte Cellulose verwendet wird.

32. Verfahren gemäss Anspruch 31, wobei acetylierte Cellulose verwendet wird.

33. Verfahren gemäss Anspruch 32, wobei Triacetylcellulose verwendet wird.

34. Verfahren gemäss Anspruch 31, wobei benzoylierte Cellulose verwendet wird.

35. Verfahren gemäss Anspruch 31, wobei Tri-(3-methylbenzyl)-Cellulose verwendet wird.

36. Verfahren gemäss Anspruch 27 oder 28, wobei das Lösungmittel der Chlorierungsstufe ein halogenierter Kohlenwasserstoff ist.

37. Verfahren gemäss Anspruch 27 oder 28, wobei das Lösungsmittel der Chlorierungsstufe eine Alkancarbonsäure ist.

38. Verfahren gemäss Anspruch 37, wobei als Alkancabonsäure Ameisensäure verwendet wird.

39. Verfahren gemäss Anspruch 37, wobei als Alkancarbonsäure Essigsäure verwendet wird.

40. Verfahren gemäss Anspruch 27 oder 28, wobei die Chlorierung bei Raumtemperatur durchgeführt wird.

41. Verfahren gemäss Anspruch 27 oder 28, wobei als Chlorierungsmittel Chlor verwendet wird.

42. Verfahren gemäss Anspruch 27 oder 28, wobei als Chlorierungsmittel Sulfurylchlorid (= $SO_2Cl_2$) verwendet wird.

43. Verfahren gemäss Anspruch 27 oder 28, wobei man in Abwesenheit einer Lewis-Säure chloriert.

44. Verfahren gemäss Anspruch 27 oder 28, wobei man als Lewis-Säure Aluminiumchlorid, Zinkchlorid oder Zinn(IV)chlorid einsetzt.

45. Verfahren gemäss Anspruch 27 oder 28, wobei man Eisen(III)chlorid als Lewis-Säure einsetzt.

46. Verfahren zur Herstellung des optisch aktiven (aS,1'R)-3-[N-(Methoxyacetyl)-N-(3-chlor-2,6-dimethylphenyl)]-amino-tetrahydro-2-furanon, dadurch gekennzeichnet, dass man das gelöste (1'R)3-[N-(methoxyacetyl)-N-(3-chlor-2,6-dimethylphenyl)]-amino-tetrahydro-2-furanon chloriert, und das entstandene (aS,1'R)-3-[N-(Methoxyacetyl)-N-(3-chlor-2,6-dimethylphenyl)]-amino-tetrahydro-2-furanon aus dem Reaktionsgemisch durch Adsorptionschromatographie vom gleichfalls entstandenen (aR,1'R)-Enantiomeren abtrennt.

47. Verfahren zur Bekämpfung pflanzenschädigender Mikroorganismen, dadurch gekennzeichnet, dass man auf die Pflanze oder deren Standort eine mikrobizid wirksame Menge von im wesentlichen (aS,1'R)-3-[N-(Methoxyacetyl)-N-(3-chlor-2,6-dimethylphenyl)]-amino-tetrahydro-2-furanon oder im wesentlichen einem Gemisch aus (aR,1'S)- und (aS,1'R)-3-[N-(Methoxyacetyl)-N-(3-chlor-2,6-dimethylphenyl)]-amino-tetrahydro-2-furanon appliziert.

48. Verfahren zur Bekämpfung pflanzenschädigender Mikroorganismen, dadurch gekennzeichnet, dass man auf die Pflanze oder deren Standort eine mikrobizid wirksame Menge von im wesentlichen einem Gemisch aus (aR,1'R)- und (aS,1'R)-3-[N-(Methoxyacetyl)-N-(3-chlor-2,6-dimethylphenyl)]-amino-tetrahydro-2-furanon appliziert.

**Patentansprüche für den Vertragsstaat: ES**

1. Verfahren zur Herstellung des Enantiomerenpaares (aR,1'S)(aS,1'R)-3-[N-(Methoxyacetyl)-N-(3-chlor-2,6-dimethylphenyl)]-amino-tetrahydro-2-furanon, dadurch gekennzeichnet, dass man das entsprechende Racemat, das aus zwei diastereomeren Enantiomerenpaaren besteht, an einer stationären festen Phase mit Hilfe einer flüssigen Phase vom (aS,1'S)(aR,1'R)-Enantiomerenpaar abtrennt.

2. Verfahren gemäss Anspruch 1, wobei die feste Phase ein Trägermaterial auf der Basis von Agarose, Sepharose, Cellulose, Polyamid oder Polyacrylamide ist.

3. Verfahren gemäss Anspruch 2, wobei das Trägermaterial benzoylierte oder acetylierte Cellulose ist.

4. Verfahren gemäss Anspruch 3, wobei Triacetylcellulose als Trägermaterial verwendet wird.

5. Verfahren gemäss Anspruch 1, wobei die feste Phase ein Trägermaterial auf der Basis von Kieselgel oder Aluminiumoxide ist.

6. Verfahren zur Herstellung des enantiomeren (aS,1'R)-3-[N-(Methoxyacetyl)-N-(3-chlor-2,6-dimethylphenyl)]-amino-tetrahydro-2-furanon, dadurch gekennzeichnet, dass man eine Lösung des Racemats bestehend aus (aR,1'S)- und (aS,1'R)-3-[N-(Methoxyacetyl)-N-(3-chlor-2,6-dimethylphenyl)]-amino-tetrahydro-2-furanon chromatographisch über eine optisch aktive feste Phase in die Enantiomeren trennt und das (aS,1'R)-Enantiomere aus der Lösung isoliert.

7. Verfahren gemäss Anspruch 6, wobei als optisch aktive feste Phase Triacetylcellulose verwendet wird.

8. Verfahren gemäss Anspruch 6, wobei als optisch aktive feste Phase Tri-(3-methylbenzoyl)-cellulose verwendet wird.

9. Verfahren zur Herstellung von (aR,1'R)(aS,1'R)-3-[N-(methoxyacetyl)-N-(3-chlor-2,6-dimethylphenyl)]-amino-tetrahydro-2-furanon, dadurch gekennzeichent, dass man (1'R)-3-[N-(methoxyacetyl)-N-(2,6-dimethylphenyl)]-amino-tetrahydro-2-furanon in einem geeigneten Lösungsmittel bei 0°—50°C in Anwesenheit oder Abwesenheit einer Lewis-Säure chloriert.

10. Verfahren zur Herstellung von (aR,1'R)(aS,1'R)-3-[N-(methoxyacetyl)-N-(3-chlor-2,6-dimethylphenyl)]-amino-tetrahydro-2-furanon, dadurch gekennzeichnet, dass man aus racemischen 3-[N-(methoxyacetyl)-N-(2,6-dimethylphenyl)]-amino-tetrahydro-2-furanon a) an einem festen Trägermaterial das (1'R)-Enantiomer abtrennt, und b) das erhaltene (1'R)-Enantiomer in einem geeigneten Lösungsmittel bei 0°—50°C in Anwesenheit oder Abwesenheit einer Lewis-Säure chloriert.

11. Verfahren gemäss Anspruch 10, wobei Silicagel oder $Al_2O_3$ als festes Trägermaterial verwendet wird.

12. Verfahren gemäss Anspruch 10, wobei ein festes Trägermaterial auf Basis von Cellulose verwendet wird.

13. Verfahren gemäss Anspruch 12, wobei acylierte Cellulose verwendet wird.

14. Verfahren gemäss Anspruch 13, wobei acetylierte Cellulose verwendet wird.

15. Verfahren gemäss Anspruch 14, wobei Triacetylcellulose verwendet wird.

16. Verfahren gemäss Anspruch 13, wobei benzoylierte Cellulose verwendet wird.

17. Verfahren gemäss Anspruch 13, wobei Tri-(3-methylbenzyl)-Cellulose verwendet wird.

18. Verfahren gemäss Anspruch 9 oder 10, wobei das Lösungmittel der Chlorierungsstufe ein halogenierter Kohlenwasserstoff ist.

19. Verfahren gemäss Anspruch 9 oder 10, wobei das Lösungsmittel der Chlorierungsstufe eine Alkancarbonsäure ist.

20. Verfahren gemäss Anspruch 19, wobei als Alkancabonsäure Ameisensäure verwendet wird.

21. Verfahren gemäss Anspruch 19, wobei als Alkancarbonsäure Essigsäure verwendet wird.

22. Verfahren gemäss Anspruch 9 oder 10, wobei die Chlorierung bei Raumtemperatur durchgeführt wird.

23. Verfahren gemäss Anspruch 9 oder 10, wobei als Chlorierungsmittel Chlor verwendet wird.

24. Verfahren gemäss Anspruch 9 oder 10, wobei als Chlorierungsmittel Sulfurylchlorid (= $SO_2Cl_2$) verwendet wird.

25. Verfahren gemäss Anspruch 9 oder 10, wobei man in Abwesenheit einer Lewis-Säure chloriert.

26. Verfahren gemäss Anspruch 9 oder 10, wobei man als Lewis-Säure Aluminiumchlorid, Zinkchlorid oder Zinn(IV)chlorid einsetzt.

27. Verfahren gemäss Anspruch 9 oder 10, wobei man Eisen(III)chlorid als Lewis-Säure einsetzt.

28. Verfahren zur Herstellung des optisch aktiven (aS,1'R)-3-[N-(Methoxyacetyl)-N-(3-chlor-2,6-dimethylphenyl)]-amino-tetrahydro-2-furanon, dadurch gekennzeichnet, dass man das gelöste (1'R)3-[N-(Methoxyacetyl)-N-(2,6-dimethylphenyl)]-amino-tetrahydro-2-furanon chloriert, und das entstandene (aS,1'R)-3-[N-(methoxyacetyl)-N-(3-chlor-2,6-dimethylphenyl)]-amino-tetrahydro-2-furanon aus dem Reaktionsgemisch durch Adsorptionschromatographie vom gleichfalls entstandenen (aR,1'R)-Enantiomeren abtrennt.

**Revendications pour les Etats contractants: AT BE CH DE FR GB GR IT LI LU NL SE**

1. Produit fongicide contenant en tant que substance active en quantité de 0,1 à 95% en poids la 3-[N-(méthoxyacétyl)-N-(3-chloro-2,6-diméthylphényl)]amino-tétrahydro-2-furannone avec une répartition irrégullière des quatre isomères possibles, caractérisé en ce que l'énantiomère (aS,1'R) déterminant l'activité est en proportion supérieure à 25% en poids, avec un véhicule approprié.

2. Produit selon la revendication 1, dans lequel la proportion de l'énantiomère (aS,1'R) est égale à 28% en poids ou plus.

3. Produit selon la revendication 1, dans lequel la proportion de l'énantiomère (aS,1'R) est égale à 40% en poids ou plus.

4. Produit selon la revendication 1, dans lequel la proportion de l'énantiomère (aS,1'R) est égale à 50% en poids ou plus.

5. Produit selon la revendication 1, dans lequel la proportion de l'énantiomère (aS,1'R) est égale à 70% en poids ou plus.

6. Produit selon la revendication 1, dans lequel la 3-[N-(méthoxyacétyl)-N-(3-chloro-2,6-diméthylphényl)]amino-tétrahydro-2-furannone consiste essentiellement (= 90% en poids ou plus) en l'énantiomère (aS,1'R).

7. Produit selon la revendication 1, caractérisé en ce que l'énantiomère (aS,1'R) déterminant l'activé représente la plus forte proportion, suivie d'une proportion égale ou inférieure, au choix, de l'énantiomère (aR,1'R) ou de l'énantiomère (aR,1'S).

8. Produit selon la revendication 7, dans lequel la substance active consiste essentiellement et un mélange de l'énantiomère (aS,1'R) et de l'énantiomère (aR,1'R).

9. Produit selon la revendication 7, dans lequel la substance active consiste essentiellement en un mélange de l'énantiomère (aS,1'R) et de l'énantiomère (aR,1'S).

10. La 3-[N-(méthoxyacétyl)-N-(3-chloro-2,6-diméthylphényl)]amino-tétrahydro-2-furannone de formule I

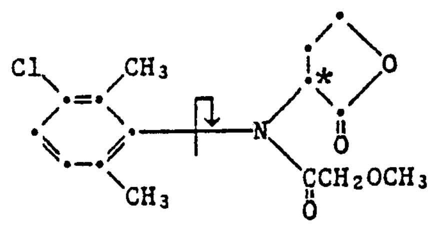

(I)

contenant l'énantiomère (aS,1'R) en proportion supérieure à 25% en poids.

11. Le composé de formule I de la revendication 10, dans lequel la proportion de l'énantiomère (aS,1'R) est d'au moins 28% en poids.

12. Le composé de formule I de la revendication 10, dans lequel la proportion de l'énantiomère (aS,1'R) est d'au moins 40% en poids.

13. Le composé de formule I de la revendication 10, dans lequel la proportion de l'énantiomère (aS,1'R) est d'au moins 50% en poids.

14. Le composé de formule I de la revendication 10, dans lequel la proportion de l'énantiomère (aS,1'R) est d'au moins 70% en poids.

15. Le composé de formule I de la revendication 10, dans lequel la proportion de l'énantiomère (aS,1'R) est d'au moins 90% en poids.

16. L'énantiomère (aS,1'R) du composé 3-[N-(méthoxyacétyl)-N-(3-chloro-2,6-diméthylphényl)]amino-tétrahydro-2-furannone.

17. Le racémate de l'(aR,1'S)- et de l'(aS,1'R)-3-[N-(méthoxyacétyl)-N-(3-chloro-2,6-diméthylphényl)]-amino-tétrahydro-2-furannone.

18. Le mélange de diastéréroisomères (aS,1'R)(aR,1'R) de la 3-[N-(méthoxyacétyl)-N-(3-chloro-2,6-diméthylphényl)]amino-tétrahydro-2-furannone.

19. Procédé de préparation de la paire d'énantiomères (aR,1'S)(aS,1'R)-3-[N-(méthoxyacétyl)-N-(3-chloro-2,6-diméthylphényl)]amino-tétrahydro-2-furannone, caractérisé en ce que l'on sépare le racémate correspondant, consistant en deux paires d'énantiomères diastéréoisomères, de la paire d'énantiomère (aS,1'S)(aR,1'R), sur une phase solide stationnaire à l'aide d'une phase liquide.

20. Procédé selon la revendication 19, dans lequel la phase solid est une matière de support à base d'agarose, de Sepharose, de cellulose, de polyamide ou de polyacrylamide.

21. Procédé selon la revendication 20, dans lequel la matière de support consiste en cellulose benzoylée ou acétylée.

22. Procédé selon la revendication 21, dans lequel on utilise la triacétylcellulose en tant que matière de support.

23. Procédé selon la revendication 21, dans lequel la phase solide est une matière de support à base de gel de silice ou d'alumine.

24. Procédé pour préparer l'énantiomère (aS,1'R) de la 3-[N-(méthoxyacétyl)-N-(3-chloro-2,6-diméthylphényl)]amino-tétrahydro-2-furannone, caractérisé en ce que l'on sépare une solution du racémate consistant en l'(aR,1'S)- et l'(aS,1'R)-3-[N-(méthoxyacétyl)-N-(3-chloro-2,6-diméthylphényl)]amino-tétrahydro-2-furannone en les énantiomères par chromatographie sur une phase solide possédant l'activé optique, et on isole l'énantiomère (aS,1'R) de la solution.

25. Procédé selon la revendication 24, dans lequel on utilise en tant que phase solide possédant l'activité optique de la triacétylcellulose.

26. Procédé selon la revendication 25, dans lequel on utilise en tant que phase solide possédant l'activité optique la tri-(2-méthylbenzoyl)-cellulose.

27. Procédé de préparation de 1'(aR,1'R)(aS,1'R)-3-[N-(méthoxyacétyl)-N-(3-chloro-2,6-diméthylphényl)]amino-tétrahydro-2-furannone, caractérisé en ce que l'on chlore la (1'R)-3-[N-(méthoxyacétyl)-N-(2,6-diméthylphényl)]amino-tétrahydro-2-furannone dans un solvant approprié à des températures de 0 à 50°, en présence ou en l'absence d'un acide de Lewis.

28. Procédé de préparation de 1'(aR,1'R)(aS,1'R)-3-[N-(méthoxyacétyl)-N-(3-chloro-2,6-diméthylphényl)]amino-tétrahydro-2-furannone, caractérisé en ce que, partant de la 3-[N-(méthoxyacétyl)-N-(2,6-diméthylphényl)]amino-tétrahydro-2-furannone racémique, a) on sépare sur une matière de support solide

l'énantiomère (1'R), et, b) on soumet l'énantiomère (1'R) à chloruration dans un solvant approprié à des températures de 0 à 50° en présence ou en l'absence d'un acide de Lewis.

29. Procédé selon la revendication 28, dans lequel on utilise en tant que matière de support solide du gel de silice ou de l'alumine.

30. Procédé selon la revendication 29, dans lequel on utilise une matière de support solide à base de cellulose.

31. Procédé selon la revendication 30, dans lequel on utilise de la cellulose acylée.

32. Procédé selon la revendication 31, dans lequel on utilise de la cellulose acétylée.

33. Procédé selon la revendication 32, dans lequel on utilise de la triacétyl-cellulose.

34. Procédé selon la revendication 31, dans lequel on utilise de la cellulose benzoylée.

35. Procédé selon la revendication 31, dans lequel on utilise de la tri-(3-méthylbenzoyl)-cellulose.

36. Procédé selon la revendication 27 ou 28, dans lequel le solvent utilisé à la chloruration est un hydrocarbure halogéné.

37. Procédé selon la revendication 27 ou 28, dans lequel le solvent utilisé à la chloruration est un acide alcanoïque.

38. Procédé selon la revendication 37, dans lequel l'acide alcanoïque est l'acide formique.

39. Procédé selon la revendication 37, dans lequel l'acide alcanoïque est l'acide acétique.

40. Procédé selon la revendication 27 ou 28, dans lequel la chloruration est effectuée à température ambiante.

41. Procédé selon la revendication 27 ou 28, dans lequel l'agent chlorant utilisé est le chlore.

42. Procédé selon la revendication 27 ou 28, dans lequel l'agent chlorant utilisé est le chlorure de sulfuryle (= $SO_2Cl_2$).

43. Procédé selon la revendication 27 ou 28, dans lequel on chlore en l'absence d'un acide de Lewis.

44. Procédé selon la revendication 27 ou 28, dans lequel on utilisé en tant qu'acide de Lewis le chlorure d'aluminium, le chlorure de zinc ou le chlorure stannique.

45. Procédé selon la revendication 27 ou 28, dans lequel on utilise en tant qu'acide de Lewis le chlorure ferrique.

46. Procédé de préparation de l'isomère optique (aS,1'R) de la 3-[N-(méthoxyacétyl)-N-(3-chloro-2,6-diméthylphényl)]amino-tétrahydro-2-furannone, caractérisé en ce que l'on acide la (1'R)-3-[N-(méthoxyacétyl)-N-(2,6-diméthylphényl)]amino-tétrahydro-2-furannone en solution et on sépare l'(aS,1'R)-3-[N-(méthoxyacétyl)-N-(3-chloro-2,6-diméthylphényl)]amino-tétrahydro-2-furannone de l'énantiomère (aR,1'R) également formé dans le mélange réaction par chromatographie d'adsorption.

47. Procédé pour combattre les microorganismes nuisibles pour les végétaux, caractérisé en ce que l'on applique sur la plante ou son habitat une quantité microbicide efficace de 3-[N-(méthoxyacétyl)-N-(3-chloro-2,6-diméthylphényl)]amino-tétrahydro-2-furannone consistant essentiellement en l'isomère (aS,1'R) ou en un mélange des isomères (aR,1'S) et (aS,1'R).

48. Procédé pour combattre les microorganismes nuisibles pour les végétaux, caractérisé en ce que l'on applique sur la plante ou son habitat une quantité microbicide efficace de 3-[N-(méthoxyacétyl)-N-(3-chloro-2,6-diméthylphényl)]amino-tétrahydro-2-furannone consistant essentiellement en un mélange des isomères (aR,1'R) et (aS,1'R).

**Revendications pour l'Etat Contractant: ES**

1. Procédé de préparation de la paire d'énantiomères (aR,1'S)(aS,1'R)-3-[N-(méthoxyacétyl)-N-(3-chloro-2,6-diméthylphényl)]amino-tétrahydro-2-furannone, caractérisé en ce que l'on sépare le racémate correspondant, consistant en deux paires d'énantiomères diastéréoisomères, de la paire d'énantiomère (aS,1'S)(aR,1'R), sur une phase solide stationnaire à l'aide d'une phase liquide.

2. Procédé selon la revendication 1, dans lequel la phase solid est une matière de support à base d'agarose, de cellulose, de Sepharose, de polyamide ou de polyacrylamide.

3. Procédé selon la revendication 2, dans lequel la matière de support consiste en cellulose benzoylée ou acétylée.

4. Procédé selon la revendication 3, caractérisé en ce que l'on utilise en tant que matière de support de la triacétylcellulose.

5. Procédé selon la revendication 1, dans lequel la phase solide est une matière de support à base de gel de silice ou d'alumine.

6. Procédé pour préparer l'énantiomère (aS,1'R) de la 3-[N-(méthoxyacétyl)-N-(3-chloro-2,6-diméthylphényl)]amino-tétrahydro-2-furannone, caractérisé en ce que l'on sépare une solution du racémate consistant en l'(aR,1'S)- et l'(aS,1'R)-3-[N-(méthoxyacétyl)-N-(3-chloro-2,6-diméthylphényl)]amino-tétrahydro-2-furannone en les énantiomères par chromatographie sur une phase solide possédant l'activé optique, et on isole l'énantiomère (aS,1'R) de la solution.

7. Procédé selon la revendication 6, dans lequel on utilise en tant que phase solide possédant l'activité optique de la triacétylcellulose.

8. Procédé selon la revendication 6, dans lequel on utilise en tant que phase solide possédant l'activité optique la tri-(2-méthylbenzoyl)-cellulose.

9. Procédé de préparation de 1'(aR,1'R)(aS,1'R)-3-[N-(méthoxyacétyl)-N-(3-chloro-2,6-diméthyl-

phényl)]amino-tétrahydro-2-furannone, caractérisé en ce que l'on chlore la (1'R)-3-[N-(méthoxyacétyl)-N-(2,6-diméthylphényl)]amino-tétrahydro-2-furannone dans un solvant approprié à des températures de 0 à 50°, en présence ou en l'absence d'un acide de Lewis.

10. Procédé de préparation de 1'(aR,1'R)(aS,1'R)-3-[N-(méthoxyacétyl)-N-(3-chloro-2,6-diméthylphényl)]amino-tétrahydro-2-furannone, caractérisé en ce que, partant de la 3-[N-(méthoxyacétyl)-N-(2,6-diméthylphényl)]amino-tétrahydro-2-furannone racémique, a) on sépare sur une matière de support solide l'énantiomère (1'R), et, b) on soumet l'énantiomère(1'R) à chloruration dans un solvant approprié à des températures de 0 à 50° en présence ou en l'absence d'un acide de Lewis.

11. Procédé selon la revendication 10, dans lequel on utilise en tant que matière de support solide du gel de silice ou de l'alumine.

12. Procédé selon la revendication 10, dans lequel on utilise une matière de support solide à base de cellulose.

13. Procédé selon la revendication 12, dans lequel on utilise de la cellulose acylée.

14. Procédé selon la revendication 13, dans lequel on utilise de la cellulose acétylée.

15. Procédé selon la revendication 14, dans lequel on utilise de la triacétyl-cellulose.

16. Procédé selon la revendication 13, dans lequel on utilise de la cellulose benzoylée.

17. Procédé selon la revendication 13, dans lequel on utilise de la tri-(3-méthylbenzoyl)-cellulose.

18. Procédé selon la revendication 9 ou 10, dans lequel le solvent utilisé à la chloruration est un hydrocarbure halogéné.

19. Procédé selon la revendication 9 ou 10, dans lequel le solvent utilisé à la chloruration est un acide alcanoïque.

20. Procédé selon la revendication 19, dans lequel l'acide alcanoïque est l'acide formique.

21. Procédé selon la revendication 19, dans lequel l'acide alcanoïque est l'acide acétique.

22. Procédé selon la revendication 9 ou 10, dans lequel la chloruration est effectuée à température ambiante.

23. Procédé selon la revendication 9 ou 10, dans lequel l'agent chlorant utilisé est le chlore.

24. Procédé selon la revendication 9 ou 10, dans lequel l'agent chlorant utilisé est le chlorure de sulfuryle (= $SO_2Cl_2$).

25. Procédé selon la revendication 9 ou 10, dans lequel on chlore en l'absence d'un acide de Lewis.

26. Procédé selon la revendication 9 ou 10, dans lequel on utilisé en tant qu'acide de Lewis le chlorure d'aluminium, le chlorure de zinc ou le chlorure stannique.

27. Procédé selon la revendication 9 ou 10, dans lequel on utilise en tant qu'acide de Lewis le chlorure ferrique.

28. Procédé de préparation de l'isomère optique (aS,1'R) de la 3-[N-(méthoxyacétyl)-N-(3-chloro-2,6-diméthylphényl)]amino-tétrahydro-2-furannone, caractérisé en ce que l'on chlore la (1'R)-3-[N-(méthoxyacétyl)-N-(2,6-diméthylphényl)]amino-tétrahydro-2-furannone en solution et on sépare l'(aS,1'R)-3-[N-(méthoxyacétyl)-N-(3-chloro-2,6-diméthylphényl)]amino-tétrahydro-2-furannone de l'énantiomère (aR,1'R) également formé dans le mélange réaction par chromatographie d'adsorption.

**Claims for the Contracting States: AT BE CH DE FR GB GR IT LI LU NL SE**

1. A fungicidal composition which contains as active ingredient 0.1 to 95% by weight of the 4 possible isomers of 3-[N-(methoxyacetyl)-N-(3-chloro-2,6-dimethylphenyl)]aminotetrahydrofuran-2-one in unequal distribution, wherein the proportion of the activity-determining (aS,1'R) enantiomer is greater than 25% by weight, together with a suitable carrier.

2. A composition according to claim 1, wherein the proportion of (aS,1'R) enantiomer is 28% by weight or more.

3. A composition according to claim 1, wherein the proportion of (aS,1'R) enantiomer is 40% by weight or more.

4. A composition according to claim 1, wherein the proportion of (aS,1'R) enantiomer is 50% by weight or more.

5. A composition according to claim 1, wherein the proportion of (aS,1'R) enantiomer is 70% by weight or more.

6. A composition according to claim 1, wherein 3-[N-(methoxyacetyl)-N-(3-chloro-2,6-dimethylphenyl)]aminotetrahydrofuran-2-one consists of preponderantly (90% by weight or more) of the (aS,1'R) enantiomer.

7. A composition according to claim 1, wherein the activity-determining (aS,1'R) enantiomer constitutes the highest proportion, followed by an equally high or lower proportion of either the (aR,1'R) enantiomer or the (aR,1'S) enantiomer.

8. A composition according to claim 7, wherein the active ingredient consists substantially of a mixture of the (aS,1'R) enantiomer and the (aR,1'R) enantiomer.

9. A composition according to claim 7, wherein the active ingredient consists substantially of a mixture of the (aS,1'R) enantiomer and the (aR,1'S) enantiomer.

10. 3-[N-(methoxyacetyl)-N-(3-chloro-2,6-dimethylphenyl)]aminotetrahydrofuran-2-one of formula I

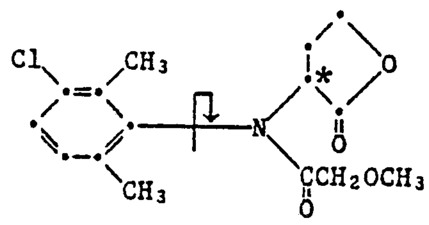

(I)

wherein the proportion of (aS,1'R) enantiomer is greater than 25% by weight.

11. The compound of formula I acording to claim 10, wherein the proportion of (aS,1'R) enantiomer is at least 28% by weight.

12. The compound of formula I acording to claim 10, wherein the proportion of (aS,1'R) enantiomer is at least 40% by weight.

13. The compound of formula I acording to claim 10, wherein the proportion of (aS,1'R) enantiomer is at least 50% by weight.

14. The compound of formula I acording to claim 10, wherein the proportion of (aS,1'R) enantiomer is at least 70% by weight.

15. The compound of formula I acording to claim 10, wherein the proportion of (aS,1'R) enantiomer is at least 90% by weight.

16. The (aS,1'R) enantiomer of the compound 3-[N-(methoxyacetyl)-N-(3-chloro-2,6-dimethylphenyl)]-aminotetrahydrofuran-2-one.

17. The racemate of (aR,1'S)- and (aS,1'R)-3-[N-(methoxyacetyl)-N-(3-chloro-2,6-dimethylphenyl)]-aminotetrahydrofuran-2-one.

18. The mixture of (aS,1'R) and (aR,1'R) diastereoisomers of 3-[N-(methoxyacetyl)-N-(3-chloro-2,6-dimethylphenyl)]aminotetrahydrofuran-2-one.

19. A process for the preparation of the pair of (aR,1'S) and (aS,1'R) enantiomers of 3-[N-(methoxyacetyl)-N-(3-chloro-2,6-dimethylphenyl)]aminotetrahydrofuran-2-one, which comprises separating the corresponding racemate consisting of two pairs of diastereoisomeric enantiomers from the pair of (aS,1'R)(aR,1'R) enantiomers on a stationary solid phase with the aid of a liquid phase.

20. A process according to claim 19, wherein the solid phase is a carrier material based on agarose, sepharose, cellulose, polyamide or polyacrylamide.

21. A process according to claim 20, wherein the carrier material is benzoylated or acetylated cellulose.

22. A process according to claim 21, wherein the triacetylcellulose is used as carrier material.

23. A process according to claim 19, wherein the solid phase is a carrier material based on silica gel or aluminium oxide.

24. A process of the preparation of the (aS,1'R) enantiomer of 3-[N-(methoxyacetyl)-N-(3-chloro-2,6-dimethylphenyl)]aminotetrahydrofuran-2-one, which comprises separating a solution of the racemate consisting of (aR,1'S)- and (aS,1'R)-3-[N-(methoxyacetyl)-N-(3-chloro-2,6-dimethylphenyl)]aminotetrahydrofuran-2-one into the enantiomers by chromatography on an optically active solid phase and isolating the (aS,1'R) enantiomer from the solution.

25. A process according to claim 24, wherein triacetylcellulose is used as optically active solid phase.

26. A process according to claim 24, wherein tris(3-methylbenzoyl)cellulose is used as optically active solid phase.

27. A process for the preparation of (aR,1'R)(aS,1'R)-3-[N-(methoxyacetyl)-N-(3-chloro-2,6-dimethylphenyl)]aminotetrahydrofuran-2-one, which comprises chlorinating (1'R)-3-[N-(methoxyacetyl)-N-(2,6-dimethylphenyl)]aminotetrahydrofuran-2-one in suitable solvent at 0°—50°C in the presence or absence of a Lewis acid.

28. A process for the preparation of (aR,1'R)(aS,1'R)-3-[N-(methoxyacetyl)-N-(3-chloro-2,6-dimethylphenyl)]aminotetrahydrofuran-2-one, which comprises a) separating the (1'R) enantiomer from racemic 3-[N-(methoxyacetyl)-N-(2,6-dimethylphenyl)]aminotetrahydrofuran-2-one on a solid carrier material and b) chlorinating the resultant (1'R) enantiomer in a suitable solvent at 0°—50°C in the presence or absence of a Lewis acid.

29. A process according to claim 28, wherein silica gel or $Al_2O_3$ is used as solid carrier material.

30. A process according to claim 28, wherein a solid carrier material based on cellulose is used.

31. A process according to claim 30, wherein acylated cellulose is used.

32. A process according to claim 31, wherein acetylated cellulose is used.

33. A process according to claim 32, wherein triacetylcellulose is used.

34. A process according to claim 31, wherein benzoylated cellulose is used.

35. A process according to claim 31, wherein tris(3-methylbenzyl)cellulose is used.

36. A process according to claim 27 or 28, wherein the solvent for the chlorination step is a halogenated hydrocarbon.

37. A process according to claim 27 or 28, wherein the solvent for the chlorination step is an alkanoic acid.

38. A process according to claim 37, wherein formic acid is used as alkanolic acid.

39. A process according to claim 37, wherein acetic acid is used as alkanolic acid.

40. A process according to claim 27 or 28, wherein the chlorination is carried out at room temperature.

41. A process according to claim 27 or 28, wherein chlorine is used as chlorinating agent.

42. A process according to claim 27 or 28, wherein sulfuryl chloride ($SO_2Cl_2$) is used as chlorinating agent.

43. A process according to claim 27 or 28, wherein chlorination is carried out in the absence of a Lewis acid.

44. A process according to claim 27 or 28, wherein aluminium chloride, zinc chloride or tin(IV) chloride is used as Lewis acid.

45. A process according to claim 27 or 28, wherein iron(III) chloride is used as Lewis acid.

46. A process for the preparation of the optically active (aS,1'R) isomer of 3-[N-(methoxyacetyl)-N-(3-chloro-2,6-dimethylphenyl)]aminotetrahydrofuran-2-one, which comprises chlorinating a solution of (1'R)-3-[N-(methoxyacetyl)-N-(2,6-dimethylphenyl)]aminotetrahydrofuran-2-one and isolating the resultant (aS,1'R) isomer of 3-[N-(methoxyacetyl)-N-(3-chloro-2,6-dimethylphenyl)]aminotetrahydrofuran-2-one from the reaction mixture by adsorption chromatography of the (aR,1'R) enantiomer also obtained.

47. A method of controlling plant-destructive microorganisms, which comprises applying to the plant or to the locus thereof a microbicidally effective amount of substantially the (aS,1'R) isomer of 3-[N-(methoxyacetyl)-N-(3-chloro-2,6-dimethylphenyl)]aminotetrahydrofuran-2-one or substantially a mixture of the (aR,1'S) and (aS,1'R) isomers of 3-[N-(methoxyacetyl)-N-(3-chloro-2,6-dimethylphenyl)]aminotetrahydrofuran-2-one.

48. A method of controlling plant-destructive microorganisms, which comprises applying to the plant or to the locus thereof a microbicidally effective amount of substantially a mixture of the (aR,1'R) and (aS,1'R) isomers of 3-[N-(methoxyacetyl)-N-(3-chloro-2,6-dimethylphenyl)]aminotetrahydrofuran-2-one.

**Claims for the Contracting State: ES**

1. A process for the preparation of the pair of (aR,1'S) and (aS,1'R) enantiomers of 3-[N-(methoxyacetyl)-N-(3-chloro-2,6-dimethylphenyl)]aminotetrahydrofuran-2-one, which comprises separating the corresponding racemate consisting of two pairs of diastereoisomeric enantiomers from the pair of (aS,1'S)(aR,1'R) enantiomers on a stationary solid phase with the aid of a liquid phase.

2. A process according to claim 1, wherein the solid phase is a carrier material based on agarose, sepharose, cellulose, polyamide or polyacrylamide.

3. A process according to claim 2, wherein the carrier material is benzoylated or acetylated cellulose.

4. A process according to claim 3, wherein the triacetylcellulose is used as carrier material.

5. A process according to claim 1, wherein the solid phase is a carrier material based on silica gel or aluminium oxide.

6. A process of the preparation of the (aS,1'R) enantiomer of 3-[N-(methoxyacetyl)-N-(3-chloro-2,6-dimethylphenyl)]aminotetrahydrofuran-2-one, which comprises separating a solution of the racemate consisting of (aR,1'S)- and (aS,1'R)-3-[N-(methoxyacetyl)-N-(3-chloro-2,6-dimethylphenyl)]aminotetrahydrofuran-2-one into the enantiomers by chromatography on an optically active solid phase and isolating the (aS,1'R) enantiomer from the solution.

7. A process according to claim 6, wherein triacetylcellulose is used as optically active solid phase.

8. A process according to claim 6, wherein tris(3-methylbenzoyl)cellulose is used as optically active solid phase.

9. A process for the preparation of (aR,1'R)(aS,1'R)-3-[N-(methoxyacetyl)-N-(3-chloro-2,6-dimethylphenyl)]aminotetrahydrofuran-2-one, which comprises chlorinating (1'R)-3-[N-(methoxyacetyl)-N-(2,6-dimethylphenyl)]aminotetrahydrofuran-2-one in suitable solvent at 0°—50°C in the presence or absence of a Lewis acid.

10. A process for the preparation of (aR,1'R)(aS,1'R)-3-[N-(methoxyacetyl)-N-(3-chloro-2,6-dimethylphenyl)]aminotetrahydrofuran-2-one, which comprises a) separating the (1'R) enantiomer from racemic 3-[N-(methoxyacetyl)-N-(2,6-dimethylphenyl)]aminotetrahydrofuran-2-one on a solid carrier metal and b) chlorinating the resultant (1'R) enantiomer in a suitable solvent at 0°—50°C in the presence or absence of a Lewis acid.

11. A process according to claim 10, wherein silica gel or $Al_2O_3$ is used as solid carrier material.

12. A process according to claim 10, wherein a solid carrier material based on cellulose is used.

13. A process according to claim 12, wherein acylated cellulose is used.

14. A process according to claim 13, wherein acetylated cellulose is used.

15. A process according to claim 14, wherein triacetylcellulose is used.

16. A process according to claim 13, wherein benzoylated cellulose is used.

17. A process according to claim 13, wherein tris(3-methylbenzyl)cellulose is used.

18. A process according to claim 9 or 10, wherein the solvent for the chlorination step is a halogenated hydrocarbon.

19. A process according to claim 9 or 10, wherein the solvent for the chlorination step is an alkanoic acid.

20. A process according to claim 19, wherein formic acid is used as alkanolic acid.

21. A process according to claim 19, wherein acetic acid is used as alkanolic acid.

22. A process according to claim 9 or 10, wherein the chlorination is carried out at room temperature.

23. A process according to claim 9 or 10, wherein chlorine is used as chlorinating agent.

24. A process according to claim 9 or 10, wherein sulfuryl chloride ($SO_2Cl_2$) is used as chlorinating agent.

25. A process according to claim 9 or 10, wherein chlorination is carried out in the absence of a Lewis acid.

26. A process according to claim 9 or 10, wherein aluminium chloride, zinc chloride or tin(IV) chloride is used as Lewis acid.

27. A process according to claim 9 or 10, wherein iron(III) chloride is used as Lewis acid.

28. A process for the preparation of the optically active (aS,1'R) isomer of 3-[N-(methoxyacetyl)-N-(3-chloro-2,6-dimethylphenyl)]aminotetrahydrofuran-2-one, which comprises chlorinating a solution of (1'R)-3-[N-(methoxyacetyl)-N-(2,6-dimethylphenyl)]aminotetrahydrofuran-2-one and isolating the resultant (aS,1'R) isomer of 3-[N-(methoxyacetyl)-N-(3-chloro-2,6-dimethylphenyl)]aminotetrahydrofuran-2-one from the reaction mixture by adsorption chromatography of the (aR,1'R) enantiomer also obtained.